# EUROPEAN PATENT APPLICATION

(11) **EP 1 273 666 A1**
(43) Date of publication of application: **08.01.2003**
(21) Application number: 01202581.3
(22) Date of filing: 04.07.2001
(51) Int. Cl.: C12Q 1/68

(54) **Method of selecting a DNA sequence with transcription modulating activity using a vector comprising an element with a gene transcription repressing activity**

(71) Applicant: Chromagenics B.V., 1018 TV Amsterdam (NL)
(72) Inventor: Otte, Arie Pieter, 1447 PN Purmerend (NL); Kruckeberg, Arthur Leo, 1092 AP Amsterdam (NL)
(74) Representative: Prins, Adrianus Willem

(57) **Abstract**

The invention is concerned with the systematic elucidation and identification of regulatory sequences. The invention provides among others screenings and detection methods with which regulatory sequences can be identified. The invention further provides regulatory sequences and use thereof in various fields such as, but not limited to protein production, diagnostics, transgenic plants and animals, and the therapeutic field.

## Description

The invention relates to the fields of medicine and cellular biology. The invention in particular relates to means and methods for regulation of gene transcription. The invention further relates to means and methods for determining whether a DNA sequence comprises a gene transcription modulating quality and/or a gene transcription repressing quality.

With the progression of the various genome projects, sequences of entire organismal genomes have become available. The flood of data has raised the interest of many investigators. One of the more noticeable discoveries was the observation that the human genome does not code for significantly more genes than the genome of simple organisms like the fruit fly. The focus of many investigators is now shifting from the identification of genes to the determination of gene expression and gene function. Examples of such technologies are DNA microarrays, functional genomics applications and proteomics. These technologies have in common that they are centered around the function and expression of coding sequences. However, while our knowledge of genes increases dramatically, the understanding of how the expression of the genes is regulated is limiting the ability to apply this rapidly increasing knowledge. This is for instance the case in the generation of transgenic plants and animals and in human gene therapy. In these applications foreign nucleic acid is typically introduced into cells to obtain expression of coding sequences. Often integration of the foreign nucleic acid into the genome of the cell is required for prolonged function of the introduced sequences. However, integration of sequences into the genome leads to unpredictability of expression because the surrounding DNA influences the transcription of the integrated sequences. This unpredictability is in part due to the fact that introduced sequences cannot be provided yet with sufficient genetic information to functionally isolate the integrated sequences from the transcription influencing effects of the surrounding DNA. In another part this is due to the fact that not enough is known about the transcription influencing effects of surrounding DNA.

The present invention is concerned with DNA sequences that comprise a capacity to influence transcription of genes *in cis.* Typically, although not necessarily, the investigated sequences do not code by themselves for a functional protein. Various sequence elements with the capacity to affect gene transcription *in cis,* have been identified. These elements range from promoters, enhancers, and silencers to boundary elements and matrix attachment regions.

The fact that so many different types of regulatory sequences have been discovered gives the impression that it is very easy to design effective expression cassettes. However, quite the contrary is true. The designing of expression cassettes is still often driven by trial and error. It is quite often possible to obtain some kind of expression of a foreign gene in a target cell or its progeny. However, very often it is difficult to predict with any kind of accuracy the level of expression or the persistence of expression that an expression cassette can display in a target cell.

The present invention provides among others means and methods for detecting and isolating new transcription regulatory elements. A method of detecting, and optionally selecting, a DNA sequence with a gene transcription-modulating quality is provided, comprising providing a transcription system with a variety of a fragment-comprising vectors, said vectors comprising i) an element with a gene-transcription repressing quality, and ii) a promoter directing transcription of a reporter gene, the method further comprising performing a selection step in said transcription system in order to identify said DNA sequence with said gene transcription modulating quality. In a preferred embodiment said fragments are located between i) said element with a gene-transcription repressing quality, and ii) said promoter directing transcription of said reporter gene. RNA polymerase initiates the transcription process after binding to a specific sequence, called the promoter, that signals where RNA synthesis should begin. A modulating quality can enhance transcription from said promoter *in cis,* in a given cell type and/or a given promoter. The same DNA sequence can comprise an enhancing quality in one type of cell or with one type of promoter, whereas it can comprise another or no gene transcription modulating quality in another cell or with another type of promoter. Transcription can be influenced through a direct effect of the regulatory element (or the protein(s) binding to it) on the transcription of a particular promoter. Transcription can however, also be influenced by an indirect effect, for instance because the regulatory element affects the function of one or more other regulatory elements. A gene transcription modulating quality can also comprise a stable gene transcription quality. With stable is meant that the observed transcription level is not significantly changed over at least 30 cell divisions. A stable quality is useful in situations wherein expression characteristics should be predictable over many cell divisions. Typical examples are cell lines transfected with foreign genes. Other examples are transgenic animals and plants and gene therapies. Very often, introduced expression cassettes function differently after increasing numbers of cell divisions or plant or animal generations. In a preferred embodiment a stable quality comprises a capacity to maintain gene transcription in subsequent generations of a transgenic plant or animal. Of course in case expression is inducible said quality comprises the quality to maintain inducibility of expression in subsequent generations of a transgenic plant or animal. Frequently, expression levels drop dramatically with increasing numbers of cell divisions. With a method of the invention it is possible to detect and optionally select a DNA sequence that is capable of at least in part preventing the dramatic drop in transcription levels with increasing numbers of cell divisions. Thus in a preferred embodiment said gene transcription modulating quality comprises a stable gene transcription quality. Strikingly, fragments comprising a DNA sequence with said stable gene transcription quality can be detected and optionally selected with a method of the invention, in spite of the fact that said method does not necessarily measure long term stability of transcription. In a preferred embodiment of the invention said gene transcription modulating quality comprises a stable gene transcription enhancing quality. It has been observed that incorporation of a DNA sequence with a gene transcription modulating quality in an expression vector with a gene of interest, results in a higher level of transcription of said gene of interest, upon integration of the expression vector in the genome of a cell and moreover that said higher gene expression level is also more stable than in the absence of said DNA sequence with a gene transcription modulating quality.

In experiments designed to introduce a gene of interest into the genome of a cell and to obtain expression of said gene of interest, the following has been observed. If together with said gene of interest also a DNA sequence with a gene transcription modulating quality was introduced, more clones could be detected that expressed more than a certain amount of gene product of said gene of interest, than when said DNA sequence was not introduced together with said gene of interest. Thus the present invention also provides a method for increasing the number of cells expressing a more than a certain level of a gene product of a gene of interest upon providing said gene of interest to the genome of said cells, comprising providing said cell with a DNA sequence comprising a gene transcription modulating quality together with said gene of interest.

The chances of detecting a fragment with a gene transcription-modulating quality vary with the source from which the fragments are derived. Typically, there is no prior knowledge of the presence or absence of fragments with said quality. In those situations many fragments will not comprise a DNA sequence with a gene transcription-modulating quality. In these situations a formal selection step for DNA sequences with said quality is introduced. This is done by selection vectors comprising said sequence on the basis of a feature of a product of said reporter gene, that can be selected for or against. For instance, said gene product may induce fluorescence or a color deposit (e.g. green fluorescent protein and derivatives, luciferase, or alkaline phosphatase) or confer antibiotic resistance or induce apoptosis and cell death.

A method of the invention is particularly suited for detecting and optionally selecting a DNA sequence comprising a gene transcription-enhancing quality. It has been observed that at least some of the selected DNA sequences, when incorporated into an expression vector comprising a gene of interest, can dramatically increase gene transcription of said gene of interest in a host cell even when the vector does not comprise an element with a gene-transcription repressing quality. This gene transcription enhancing quality is very useful in cell lines transfected with foreign genes or in transgenic animals and plants.

Said transcription system can be a cell free *in vitro* transcription system. With the current expertise in automation such cell free systems can be accurate and quick. However, for the present invention said transcription system preferably comprises host cells. Using host cells warrants that fragments are detected and optionally selected with activity in cells.

An element with a gene transcription repressing quality will, in a method of the invention, repress transcription from a promoter in the transcription system used. Said repression does not have to lead to undetectable expression levels. Important is that the difference in expression levels in the absence or presence of repression is detectable and optionally selectable. In a preferred embodiment gene-transcription repression in said vectors results in gene-transcription repressing chromatin. In this preferred embodiment DNA sequences can be detected, and optionally selected that are capable of at least in part counteracting the formation of gene-transcription repressing chromatin. In one aspect a DNA sequence capable of at least in part counteracting the formation of gene-transcription repressing chromatin comprises a stable gene transcription quality. In a preferred embodiment the DNA sequence involved in gene-transcription repression is a DNA sequence that is recognized by a protein complex and wherein said transcription system comprises said complex. Preferably said complex comprises a heterochromatin-binding protein comprising HP1, a Polycomb-group (Pc-G) protein, a histone deacetylase activity or MeCP2 (methyl-CpG-binding protein). Many organisms comprise one or more of these proteins. These proteins frequently exhibit activity in other species as well. Said complex can thus also comprise proteins from two or more species. The mentioned set of known chromatin-associated protein complexes are able to convey long-range repression over many base pairs. The complexes are also involved in stably transferring the repressed status of genes to daughter cells upon cell division. Sequences selected in this way are able to convey long-range anti-repression over many base pairs (van der Vlag et al., 2000).

The vector used can be any vector that is suitable for cloning DNA and that can be used in a transcription system. When host cells are used it is preferred that the vector is an episomally replicating vector. In this way, effects due to different sites of integration of the vector are avoided. DNA elements flanking the vector at the site of integration can have effects on the level of transcription of the promoter and thereby mimic effects of fragments comprising DNA sequences with a gene transcription modulating quality. In a preferred embodiment said vector comprises a replication origin from the Epstein-Barr virus (EBV), OriP, and a nuclear antigen (EBNA-1). Such vectors are capable of replicating in many types of eukaryotic cells and assemble into chromatin under appropriate conditions.

In another aspect the invention provides a DNA sequence comprising i) a DNA sequence isolated from a plant or vertebrate, or derivatives thereof, or ii) a synthetic DNA sequence or one constructed by means of genetic engineering, which DNA sequence is a repression-inhibiting sequence which, by the method according to the present invention can be detected, selected and optionally cloned. In another aspect the invention provides a DNA sequence comprising i) a DNA sequence isolated from a plant or vertebrate, or derivatives thereof, or ii) a synthetic DNA sequence or one constructed by means of genetic engineering, which DNA sequence is detected, selected and optionally cloned by means of the method of the invention. Preferably said DNA sequence comprises a sequence as depicted in Table 4A or a functional homologue thereof. A functional homologue of a sequence as depicted in Table 4 is a sequence derived with the information given in Table 4 (be it table 4A or table 4B). For instance, a sequence that can be derived from a sequence in Table 4 by deleting, modifying and/or inserting bases in or from a sequence listed in Table 4, wherein said derived sequence comprises the same activity in kind, not necessarily in amount, of a sequence as depicted in Table 4. A functional homologue is further a sequence comprising a part from two or more sequences depicted in Table 4. A synthetic DNA sequence is a sequence that is not derived directly or indirectly from a sequence present in an organism. For instance a sequence comprising a drosophila scs or scs' sequence is not a synthetic sequence, even when the scs or scs' sequence was artificially generated.

In one aspect the invention is concerned with increasing knowledge of higher order gene regulation and with means and methods for utilizing this knowledge. Whereas elements, such as classical promoters and enhancers, have been characterized that direct and regulate transcription of single genes, higher order regulatory elements that govern the gene transcription capabilities of entire chromosome regions have as yet received little attention. Much of our knowledge regarding such higher order elements comes from the study of embryogenesis. During embryogenesis cells become committed to different developmental pathways. Once committed, cells rarely change their fates, even after many cell divisions.

It has become increasingly clear that the stable transmission of cell type specific gene transcription patterns is not dependent on the context of a promoter, but is instead mediated by changes in the structure of the DNA and associated proteins, termed chromatin. Gene regulation at the chromosomal level involves modifications of DNA (e.g. methylation), histones, (e.g. acetylation and/or methylation), and long-range interactions between distant chromosomal elements.

The chromatin template is a highly condensed complex of DNA, histones, and non-histone proteins, which is able to package the entire genome into the nucleus and simultaneously allow the appropriate transcription of specific genes. The eukaryotic chromosome is not a uniform template for the activation of gene transcription. Different types of chromatin and chromatin regions can be distinguished, which differentially affect gene transcription. The so-called heterochromatin regions identify 'closed' chromatin structures whereas euchromatin is associated with a more diffuse and 'open' chromatin structure. The euchromatin region can be subject to structural changes, resulting in more or less condensed structures, referred to as facultative heterochromatin and euchromatin. The formation of facultative euchromatin or heterochromatin is believed to represent the underlying mechanism of chromatin-mediated gene regulation, keeping genes in an active or a repressed state, in a cell type specific manner.

In all eukaryotes several chromatin-associated protein complexes have been identified that are involved in the maintenance of cell type specificity, one of which is the Polycomb group (PcG) complex. The PcG complex is involved in the stable repression of genes, in which changes in chromatin structure are believed to play an important role. Similarly, a second class of proteins, named the trithorax group (TrG), has been identified that counteracts the action of the PcG proteins. TrG proteins are involved in the maintenance of gene gene transcription. Based on their respective modes of action, PcG and TrG proteins therefore represent a cellular memory system that is important for the heritable transmission of gene transcription patterns.

How PcG and TrG complexes are associated with their target genes is still unclear. Genetic studies have characterized cis-acting regulatory sequences that maintain transcriptionally inactive states of genes. The silencing mediated by these cis-acting regulatory sequences is dependent on the presence of functional PcG proteins, and hence these sequences have been termed PcG response elements (PREs). Sequences have been identified that are involved in PcG mediated repression of chromatin. As yet however, (in both vertebrates or plants) complete PREs comprising all sequence information required to mediate repression of chromatin have not been found.

In addition, it has, as yet, not been possible to study sequences with long range repression capabilities in a coherent way. This is for a large part due to the inability to systematically screen for such long-range acting sequences. In one aspect the invention provides means and methods for systematically detecting such sequences in DNA. In one embodiment the invention provides a method for identifying a DNA sequence with a gene transcription repressing quality comprising,
- providing a collection of test nucleic acids,
- generating a collection of expression vectors comprising test nucleic acids and a first reporter gene under transcriptional control of a promoter,
- providing cells with said collection of expression vectors,
- selecting a cell or vector-containing progeny thereof, wherein transcription of said first reporter gene is repressed, and
- identifying said test nucleic acid in said cell. Said identified test nucleic acid comprises the capacity to repress said promoter function and thus comprises a gene transcription repressing quality. Preferably, said identified test nucleic acid is also retreived and cloned. Said quality comprises at least in part, the capacity to reduce the level of transcription from said promoter when physically linked to said promoter, compared to the level in the absence of the DNA sequence with said quality. In a preferred embodiment said gene transcription repressing quality comprises a gene transcription repressing chromatin quality. I.e. wherein said reduction of the level of transcription is the result of chromatin having a gene transcription repressing configuration. This configuration preferably encompasses said promoter. However, it is also possible that said configuration encompasses an enhancer or the like thereby at least in part inactivating the transcription enhancing effect of said enhancer on said promoter. In a particularly preferred embodiment said DNA sequence with a gene transcription repressing chromatin quality comprises a polycomb-group-like responsive element.

Using the above mentioned method it is possible to retrieve several nucleic acid sequences comprising the capacity to reduce the level of transcription from a promoter and thus nucleic acid sequences comprising a gene transcription repressing quality. Sequences with analogous function can be compared with each other for sequence similarities such that one or more consensus sequences for elements with a gene transcription repressing quality such as polycomb-group-like responsive elements, can be deduced. Moreover, considering that entire sequences of organismal genomes are known and more will follow shortly, it is possible to screen these genomes or parts thereof, and predict the occurrence of these sequences in the genome. Knowledge of the occurrence and localization of DNA sequences comprising gene transcription-modulating qualities and/or gene transcription repressing qualities in the genome will greatly increase our knowledge of higher order regulation of gene transcription in the genome.

A Polycomb-group response element is an element that is capable of repressing the transcription from a promoter in response to the direct and/or indirect interaction of one or more Polycomb group proteins with said element. A polycomb-group-like response element is a Polycomb-group response element or alternatively it is an element capable of repressing the transcription of a promoter upon the direct and/or indirect interaction of one or more proteins with said element, wherein said one or more proteins do not belong to the Polycomb-group, but wherein as a result of said interaction gene transcription repressing chromatin is formed. Examples of such proteins are chromatin-associated proteins such as heterochromatin proteini (HP1) (Eisenberg et al., 1990) Another chromatin-associated protein that represses gene activity is methyl-CpG-binding protein, MeCP2 (Nan et al., 1997). In a preferred embodiment a polycomb-group-like responsive element of the invention comprises the capacity to repress transcription of a promoter over long distances, preferably over more than 2000 base pairs (van der Vlag et al., 2000).

A collection of test nucleic acids can be generated in many ways. Using artificial sequences as test nucleic acids it is possible to obtain a consensus sequence for a gene transcription repressing quality. Different qualities can comprise different consensus sequences. Preferably said collection is generated from chromosomal DNA. In this way a gene transcription repressing quality comprising a sequence occurring naturally in the chromosome is found. This has the advantage that the location of these qualities in the chromosome can be determined whereupon their influence on higher order gene transcription in said location can be resolved.

A reporter gene is a gene encoding an expression product of which the presence can be detected directly or indirectly in a cell. In methods for detecting a gene transcription repressing quality, the transfer of an expression vector into a cell will lead to expression of said reporter gene. However, in case test nucleic acid comprises a gene transcription repressing quality, such as a polycomb-group-like responsive element, expression will be repressed in said cell thereby leading to an at least in part reduced expression of said reporter gene. The presence or absence of a nucleic acid capable of repressing transcription of said promoter can thus be detected by detecting said expression product in said cell, whereby reduced detection or no detection indicates the presence of a gene transcription repressing quality. A reporter gene can encode a fluorescent reporter protein. Reduced expression can then be detected by fluorometric means for instance in a flow cytometer. Cells displaying no or low fluorescence can be sorted using a fluorescence activated cell sorter and the expression vector and/or the test nucleic acid isolated, for instance by means of an amplification reaction. Preferably, said first reporter gene comprises a selectable reporter gene, the expression of which directly or indirectly provides said cell with at least a growth disadvantage over cells not expressing or expressing low levels of said first reporter gene. In cases where DNA sequences with a gene-transcription repressing quality are screened for, expression of said first reporter gene is, preferably, directly or indirectly toxic to said cell. Non-limiting examples of such a toxic expression product is ricin or toxic variants thereof. In another example, said first reporter gene encodes an apoptosis inducing gene product. Preferably said apoptosis inducing gene product comprises adenovirus 13S E1A or a functional equivalent thereof (Breckenridge and Shore, 2000). In another embodiment said apoptosis inducing gene product comprises apoptin or a functional equivalent thereof (Pietersen and Noteborn, 2000).

Another example is a gene encoding a so-called suicide product such as the herpes simplex virus thymidine kinase (HSV-tk). Addition of gancyclovir to cultures of cells expressing HSV-tk will result in the formation of a toxic substance in these cells and therefore kill these cells. In a particularly preferred embodiment said suicide gene comprises cytosine deaminase. Cytosine deaminase converts cytosine to uracil. This enzyme activity is found in prokaryotes and lower eukaryotes, but is absent in higher eukaryotes. The gene is used as a metabolic suicide gene in combination with the prodrug 5-fluorocytosine (5-FC). Cytosine deaminase is able to convert the non-toxic 5-FC into 5-fluorouracil, which kills cells by disrupting DNA synthesis, thereby triggering apoptosis (Mullen et al., 1992; Wei and Huber, 1996).

A promoter controlling transcription of said first reporter gene can be any promoter that is active, or can be activated in said cells. By selecting a particular promoter, it is possible to select a gene transcription repressing quality such as a polycomb-group-like responsive element capable of repressing transcription of said particular promoter. in this way it is possible to select qualities that specifically repress the class of promoters said particular promoter belongs to. In a preferred embodiment said promoter comprises a promoter of which the activity can be induced upon providing a cell comprising said promoter with a signal. Such an inducible promoter preferably comprises a tetracycline responsive promoter. The signal here being tetracycline, doxycycline and equivalent compounds. Such promoters can also be adapted for tetracycline responsiveness in eukaryotic cells (Yin et al., 1996). Promoters and transacting molecules are available that either induce or repress expression of a gene, upon the addition of tetracycline or equivalents thereof.

Cells transfected with an expression vector of the invention can, with a typically low frequency and for reasons not associated with the presence of a DNA sequence with a gene transcription repressing quality, not express detectable amounts of expression product of said first reporter gene. This can for instance be due to a recombination event disrupting the coding sequence of said first reporter gene. In a preferred embodiment of the invention said collection of expression vectors further comprises a second reporter gene. Expression of said second reporter gene is preferably under the control of a second promoter. Methods for the detection of expression of an expression product of said second reporter gene can be used to confirm the expression repressing activity of said test nucleic acid, thereby at least in part reducing the number of cells falsely not expressing said first reporter gene. In a preferred embodiment said second reporter gene is used to select for cells comprising an expression cassette. In this way, cells not comprising said expression cassette can easily be disregarded. To this end said expression product of said second reporter gene preferably comprises a positive dominant selectable reporter gene. Preferably, said positive dominant selectable reporter gene encodes an expression product capable of conferring resistance to an otherwise toxic compound. Non-limiting examples are G418 resistance and hygromycin resistance.

Considering that a gene transcription repressing quality can suppress transcription it is preferred that in this embodiment an expression vector further comprises at least one DNA sequence with a gene transcription modulating quality, capable of counteracting transcription repressing effects of a DNA sequence with a gene transcription repressing quality. The placement of said transcription counteracting element in the expression vector is preferably such that it effectively interferes with a reducing effect of said gene transcription repressing quality could have on the level of transcription of said second reporter gene. In a preferred embodiment said DNA sequence with a gene transcription modulating quality functionally separates the expression cassettes comprising said first and said second reporter gene. Preferably, said second reporter gene (and promoter controlling transcription of said second reporter gene) is flanked by DNA sequences with a gene transcription modulating quality. Examples of DNA sequences with a gene transcription modulating quality are the so-called STAR elements listed in Tables 1 and 2.

Methods of the invention result in the cloning and identification of a number of elements comprising a gene transcription modulating and/or a gene transcription repressing quality. Such an element may contain irrelevant nucleic acid that is not instrumental in performing said quality, for instance not involved in the formation of gene-transcription repressing chromatin. Functional sequences in such elements can be delineated by various methods known in the art. In one embodiment deletions and/or substitutions are made in a DNA sequence with a gene transcription modulating or a gene transcription repressing quality. DNA that is modified in such a way, is tested for activity in a method of the invention. This can be done using a single modified nucleic acid or by generating a collection of test nucleic acids comprising said modified nucleic acid. Elucidation of functional sequences within DNA sequences of the invention enables the elucidation of consensus sequences for elements with a gene transcription modulating and/or a gene transcription repressing quality. Considering that there are several polycomb-group-like complexes that each comprise different functionalities and expression patterns, it is anticipated that more than one type of consensus sequence is found with a method of the invention. Analogously it is anticipated that more than one type of consensus sequence is found for an element comprising a gene transcription modulating quality. The invention thus further provides a library of isolated and/or recombinant nucleic acids comprising gene transcription modulating and/or gene transcription repressing qualities such as polycomb-group-like response elements. In one embodiment said library comprises isolated and/or recombinant nucleic acids comprising the same consensus sequence. In a preferred embodiment said library comprises more than one type of consensus sequence. Said library can be used for instance for determining whether a given DNA molecule comprises a DNA modulating quality. In a preferred embodiment said library comprises essentially all elements with a gene transcription enhancing function, elements comprising a stable gene transcription quality and/or elements with a gene transcription repressing quality such as polycomb-group-like response elements, of a chromosome. Together with knowledge on the location of these elements on a chromosome this allows a person skilled in the art to generate a prediction for higher order regulation of gene expression of genes naturally present on said chromosome and for genes (foreign nucleic acid) introduced into said chromosome by recombinant means. Such a prediction can be used for example to select a suitable candidate location on said chromosome for the insertion of foreign DNA. A suitable location can be a location expected to be specifically expressed in a certain cell, cell type and/or tissue. Preferably, said chromosome comprises chromosome 21 or chromosome 22. In a particularly preferred embodiment all DNA sequences comprising a gene transcription modulating or a gene transcription repressing quality in a cell, are in the library. In this embodiment the entire genome can be used for the prediction of a suitable candidate location. In one embodiment said library has been generated in different cell lines of species ranging from plants to human. In different cell lines and/or species different proteins (or protein complexes) capable of interacting with DNA sequences with a gene transcription repressing quality, will be expressed, resulting in different DNA elements with a gene transcription repressing quality. Similarly different proteins that interact directly or indirectly with DNA sequences comprising a gene transcription modulating quality will be expressed. Therefore the make-up of the library is cell-type dependent and dependent on the presence of the relevant proteins. This is also the case with polycomb-group-like response elements. If HP1 is expressed in cell type one, elements depending on HP1 will be detected by method of invention. If HP1 is not expressed in cell type two, method of invention will not detect the element that has been retrieved from cell type one.

In one aspect of the invention said library comprises at least one element capable of at least in part counteracting the formation of gene-transcription repressing chromatin. Together with knowledge of the location of DNA sequences with a gene transcription repressing quality on a chromosome or genome, knowledge of the location of such counteracting elements allows more accurate prediction of higher order regulation of gene transcription of (inserted) genes in said chromosome or genome. Preferably said library further comprises other transcription regulatory elements such as enhancers and silencers. Although such sequences have limited influence on higher order gene regulation, information on the location of such other sequences further increases the accuracy of the prediction on suitable locations in the genome for the expression of foreign sequences introduced therein. Preferably, said library comprises essentially all DNA sequences comprising a gene transcription modulating quality and/or all other regulatory sequences of a chromosome.

Considering that already a chromosome typically consists of several tens of millions of bases, it is preferred that the information that the library can give on higher order gene regulation is incorporated into an at least partially automated system.

Another use of a library of the invention is the generation of a prediction on transcription of genes upon targeted modification of sequences on a chromosome such that "higher order" regulatory sequences are mutated. For instance, one or more polycomb-group-like responsive elements of the invention, and/or other regulatory elements on said chromosome can be mutated. This is expected to change the transcription levels of the genes that are in the vicinity of the polycomb-group-like responsive elements and/or other expression modulating elements.

Yet another use of a library or system of the invention is the prediction of gene expression resulting from mutations in the genome. In cases where a mutation results in altered gene transcription, detection of such altered gene transcription can indicate the presence of said naturally occurring mutation. This approach is useful for instance in limiting the number of sequences or proteins to be tested in a diagnostic assay. This is particularly important in microarray approaches because in these approaches the number of expressed sequences to be tested for, is limited by the number of sequences that an array can maximally hold. With means and methods of the invention it is possible to limit the number of sequences to be tested in microarray approaches.

Yet another use of a system or library of the invention is the discovery of drug targets. Regulatory elements, be they "higher order" elements or not, function because of the protein (complexes) that can bind to them. A system of the invention can be used to determine whether targeting of drugs to interfere with the binding or function of a particular protein (complex) holds promise for the alteration of expression of a particular gene.

The invention further provides a DNA sequence comprising a gene-transcription repressing quality obtainable by a method of the invention. In a preferred embodiment said DNA sequence comprising a gene-transcription repressing quality is derived from a vertebrate of a plant. More preferably, said DNA sequence comprising a gene-transcription repressing quality comprises a sequence according to table 4 B, or a functional homologue thereof. It is also possible to provide a DNA construct with a DNA sequence of the invention, or to modify such a DNA sequence. In a preferred embodiment a DNA construct is providid comprising a promoter operably linked with a nucleic acid of interest. Preferably, the amount of activity of a quality of said DNA sequence with a gene transcription modulating and/or repressing quality, is dependent on the orientation of said DNA sequence in said construct, compared to said promoter. Preferably said gene transcription modulating and/or repressing quality is dependent on the presence of a signal. Preferably, said signal comprises a DNA binding protein. Preferably, said signal comprises a human immuno-deficiency virus TAT protein.

One of the uses of a DNA sequence comprising a gene transcription modulating quality or a gene transcription repressing quality is of course the regulation of transcription of a gene of interest. Transcription of a gene of interest can be altered by altering sequences in the vicinity of said gene such that a DNA sequence with said quality is provided or removed. Specific expression characteristics can be designed by combining (parts of) DNA sequences with a gene transcription modulating and/or a gene transcription repressing quality. For instance, duplication of a sequence with a stable gene transcription quality in an expression vector will lead to improved stability of expression in a target cell or progeny upon introduction of said vector in said target cell. By combining DNA sequences with gene transcription modulating qualities altered gene transcription modulating qualities can be generated either in kind or amount or both.

It is also possible to design DNA sequences with a desired gene transcription modulating and/or a gene transcription repressing quality. DNA binding proteins together with other proteins and DNA sequences determine qualities of the DNA sequence. It is possible to insert one or more other protein binding DNA sequences into a DNA sequence with a quality. By allowing binding of the binding protein(s) it is possible to interfere with, or direct, the quality, thus allowing the generation of DNA sequences with designer qualities. It is of course also possible to remove protein binding sites from a DNA sequence with a particular gene transcription modulating and/or a gene transcription repressing quality thereby altering the quality of the resulting DNA sequences. The combination of addition and removal is also possible. Particular gene transcription modulating and/or gene transcription repressing qualities can be selected for by tuning detection methods described in the present invention. It is for instance possible to synthesize DNA sequences with inducible gene transcription modulating and/or gene transcription repressing qualities. By for instance including TAT-binding elements in a DNA sequence comprising a gene-transcription repressing quality, it is possible to at least in part inactivate the gene-transcription repressing quality in a cell comprising TAT. Similarly there are DNA binding proteins available that only bind to their target sequence in the absence or presence of a signal. Non-limiting examples of such proteins are the TET-repressor and the various mutations thereof, the lac-repressor, steroid hormone receptors, the retinoic acid receptor, and derivatives. It is possible for instance to design a DNA sequence with a cell type specific gene transcription modulating and/or gene transcription repressing quality. For instance, in case of the above mentioned TAT example. The referred to DNA sequence can be made specific for HIV infected cells that express TAT. Alternatively, The DNA sequence can be made specific for a protien complex that is expressed in a cell type specific fashion.

Expression constructs comprising a DNA sequence comprising a gene transcription modulating and/or gene transcription repressing quality are suitable for obtaining expression from said construct in cells comprising more than one copy of said expression construct. Also when the expression construct is present in the genome of said cell and, also when the expression cassette is present in more than one copy in said cell. Moreover, they even work when integrated into the same position in more than one copy.

The term STAR or STAR element, as used in the examples listed below, refers to a DNA sequence comprising one or more of the mentioned gene transcription modulating qualities. The term SINC or SINC element as listed below refers to a DNA sequence comprising one or more of the mentioned gene transcription repressing qualities. The term "DNA sequence" as used herein does, unless otherwise specified, not refer to a listing of specific ordering of bases but rather to a physical piece of DNA. A transcription quality with reference to a DNA sequence refers to an effect that said DNA sequence has on transcription of a gene of interest. "Quality" as used herein refers to detectable properties or attributes of a nucleic acid or protein in a transcription system.

### Examples

### Example 1. Methods to isolate STAR and SINC elements

### Materials and methods

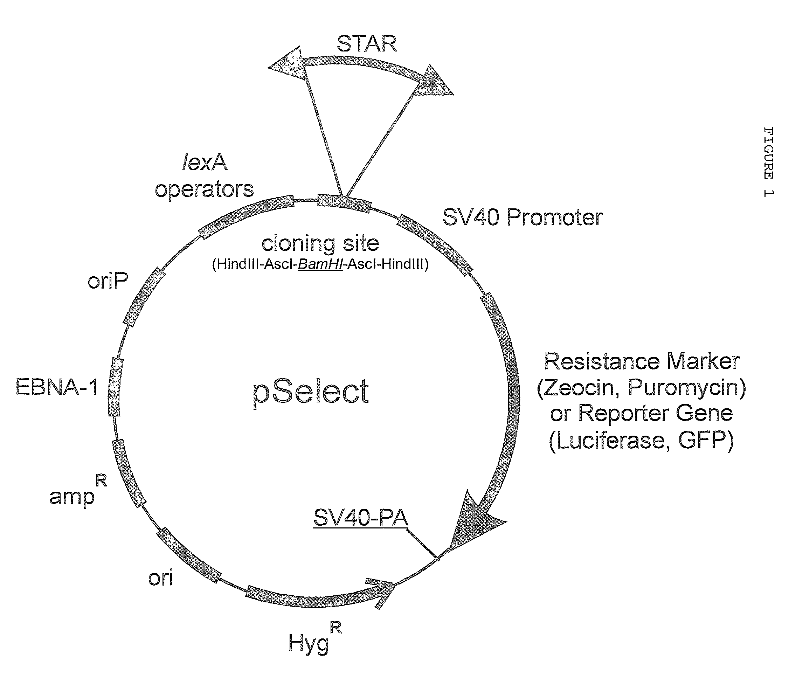

Plasmids and strains. The selection vector for STAR elements, pSelect-SV40-zeo ("pSelect", Figure 1) is constructed as follows: the pREP4 vector (Invitrogen V004-50) is used as the plasmid backbone. It provides the Epstein Barr oriP origin of replication and EBNA-1 nuclear antigen for high-copy episomal replication in primate cell lines; the hygromycin resistance gene with the thymidine kinase promoter and polyadenylation site, for selection in mammalian cells; and the ampicillin resistance gene and colE1 origin of replication for maintenance in *Escherichia coli.* The vector contains four consecutive LexA operator sites between *Xba*I and *Nhe*I restriction sites (Bunker and Kingston, 1994). Embedded between the LexA operators and the *Nhe*I site is a polylinker consisting of the following restriction sites: *Hin*dIII-*Asc*I*-Bam*HI*-Asc*I*-Hind*III. Between the *Nhe*I site and a *Sal*I site is the zeocin resistance gene with the SV40 promoter and polyadenylation site, derived from pSV40/Zeo (Invitrogen V502-20); this is the selectable marker for the STAR screen.

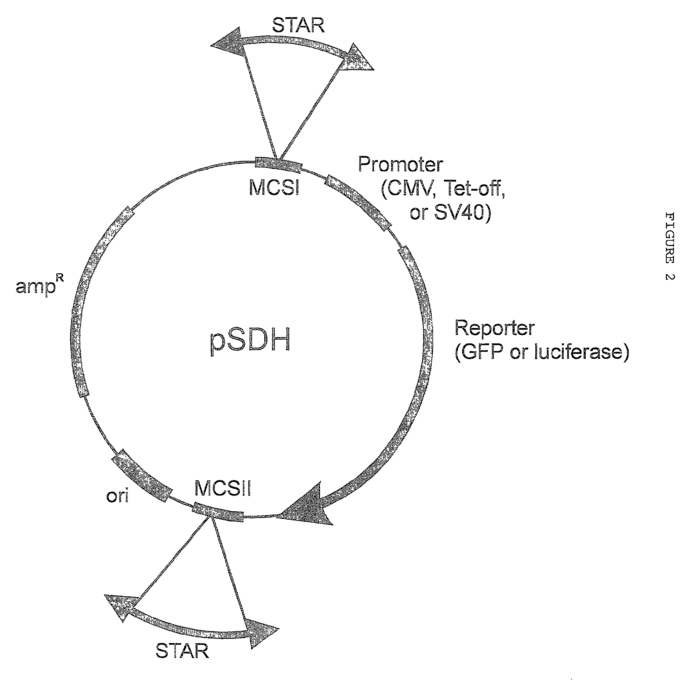

The pSDH vector (Figure 2) is constructed as follows: The luciferase reporter gene from pGL3-Control (Promega E1741) is amplified by PCR and inserted into *Sac*II/*Bam*HI-digested pUHD10-3 (Gossen and Bujard, 1992). This places luciferase under control of the Tet-Off promoter, and upstream of the SV40 polyadenylation signal. Multiple cloning sites are introduced by PCR, upstream of the Tet-Off promoter (MCSI, *Xho*I*-Not*I-*Eco*RI*-SalI*) and downstream of the polyadenylation signal (MCSII, *Nhe*I*-Bgl*II*-Eco*RV*-Hind*III). Gene libraries are constructed by *Sau*3AI digestion of human genomic DNA, either purified from placenta (Clontech 6550-1) or carried in bacterial/P1 (BAC/PAC) artificial chromosomes. The BAC/PAC clones contain genomic DNA from the 1q12 cytogenetic region (clones RP1154H19 and RP3328E19) or from the HOX cluster of homeotic genes (clones RP1167F23, RP1170019, and RP11387A1). The DNAs are size-fractionated, and the 0.5 - 2 kb size fraction is ligated into *Bam*HI-digested pSelect vector, by standard techniques (Sambrook et al., 1989).

The construction of the host strains has been described (van der Vlag et al., 2000). Briefly, they are based on the U-2 OS human osteosarcoma cell line (American Type Culture Collection HTB-96). U-2 OS is stably transfected with the pTet-Off plasmid (Clontech K1620-A), encoding a protein chimera consisting of the Tet-repressor DNA binding domain and the VP16 transactivation domain. The cell line is subsequently stably transfected with fusion protein genes containing the LexA DNA binding domain, and the coding regions of either HP1 or HPC2 (two Drosophila Polycomb group proteins that repress gene expression when tethered to DNA). The LexA-repressor genes are under control of the Tet-Off transcriptional regulatory system (Gossen and Bujard, 1992).

Library screening and STAR element characterization. The gene libraries in pSelect are transfected into the U-2 OS/Tet-Off/LexA-repressor cell line by calcium phosphate precipitation (Graham and van der Eb, 1973; Wigler et al., 1978) as recommended by the supplier of the transfection reagent (Life Technologies). Transfected cells are cultured under hygromycin selection (25 µ g/ml) and tetracycline repression (doxycycline, 10 ng/ml) for 1 week (50% confluence). Then the doxycycline concentration is reduced to 0.1 ng/ml to induce the LexA-repressor genes, and after 2 days zeocin is added to 250 µ g/ml. The cells are cultured for a further 4-5 weeks, until the control cultures (transfected with empty pSelect) are killed by the zeocin.

Zeocin-resistant colonies from the library transfection are propagated, and plasmid DNA is isolated and rescued into *E. coli* by standard techniques (Sambrook et al., 1989). The candidate STAR elements in the rescued DNA are analyzed by restriction endonuclease mapping (Sambrook et al., 1989), DNA sequence analysis (Sanger et al., 1977), and for STAR activity (zeocin resistance) after re-transfection to U-2 OS/Tet-Off/LexA-repressor and lowering the doxycycline concentration.

Candidate STAR elements that have DNA sequence corresponding to known sequence in the human genome are identified by BLAST searches (Altschul et al., 1990) of the human genome database (http://www.ncbi.nlm.nih.gov/genome/seq/HsBlast.html 20 June 2001). The chromosomal locations of the elements are recorded, along with the proportion of repetitive DNA and the identity of adjacent genes.

Those candidates that show STAR activity upon re-transfection are characterized further by subcloning the STAR fragment into the pSDH plasmid and stable integration in U-2 OS chromosomal DNA. pSDH plasmids are co-transfected into U-2 OS cells with pBABE-puro (Morgenstern and Land, 1990), and selected for puromycin resistance. Per STAR element, populations of approximately 30 individual clones are isolated and cultured. The clones are periodically assayed for luciferase activity according to the manufacturer's instructions (Roche 1669893).

### Results

STAR element functional characterization. The screens of human genomic DNA and of the HOX and 1q12 loci yielded 17 bona fide STAR elements. The criteria are that (1) the elements displayed STAR activity upon re-transfection of the pSelect-based clones into the host U-2 OS human osteosarcoma cell line (indicating that the anti-repressor activity expressed in the initial screen is plasmid-specific and not due to artefactual changes in the host cells); (2) the elements contain DNA sequence that matches sequence in the human genome sequence database (indicating that the clone does not contain contaminating DNA sequence, from e.g. bacterial or vector sources).

The STAR elements are sub-cloned into the pSDH plasmid and integrated into the host cell genome. Expression of the reporter genes is assayed in populations of stable transfectants to demonstrate the ability of the STAR elements to protect reporter genes from silencing after integration at random into the genome. This provides information (1) on the proportion of clones which display high expression, and (2) on the degree of over-expression elicited by the STAR elements.

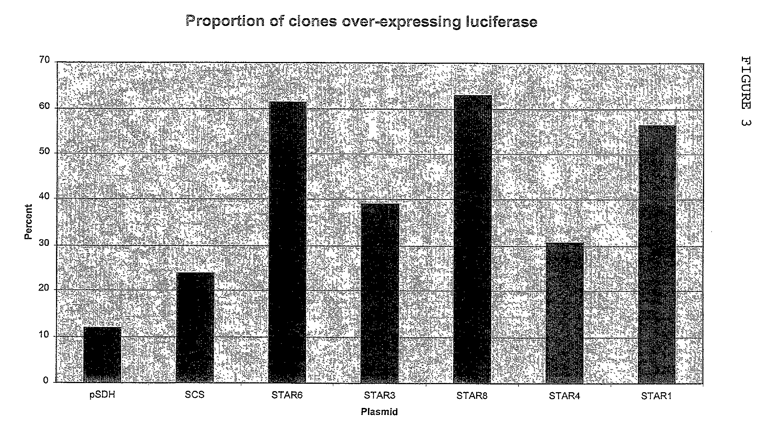

Expression of the luciferase reporter gene by a clone is considered significant if it is two-fold above the average level for the plasmids containing no STAR elements (the reference level). For all plasmids a distribution in expression level is observed among the clones: from no expression to expression significantly above the reference level, and from few over-expressers to many over-expressers. Superior STAR activity is manifested by plasmids that result in many over-expressing clones, including some highly over-expressing clones. Results from a representative experiment are shown in Table 1, and in Figures 3 - 5:

The results indicate that the human STAR elements which are tested yield a much higher proportion of over-expressing clones than the unprotected reporter gene, or the reporter gene protected by the *Drosophila* SCS element (Kellum and Schedl, 1992). Furthermore, the degree of over-expression by these plasmids is much greater from the STAR-protected reporter gene than the unprotected or SCS-protected reporter.

STAR element sequence and genomic position data. Table 2 lists the chromosomal locations of each of the 17 STAR elements, as well as the identity of nearby genes and the repetitive DNA content of the elements. The STAR elements are distributed over a number of chromosomes. They are diverse in their actual DNA sequence and repetitive DNA content, and display various degrees of association with neighboring genes.

### SINC element screen

### Materials and methods

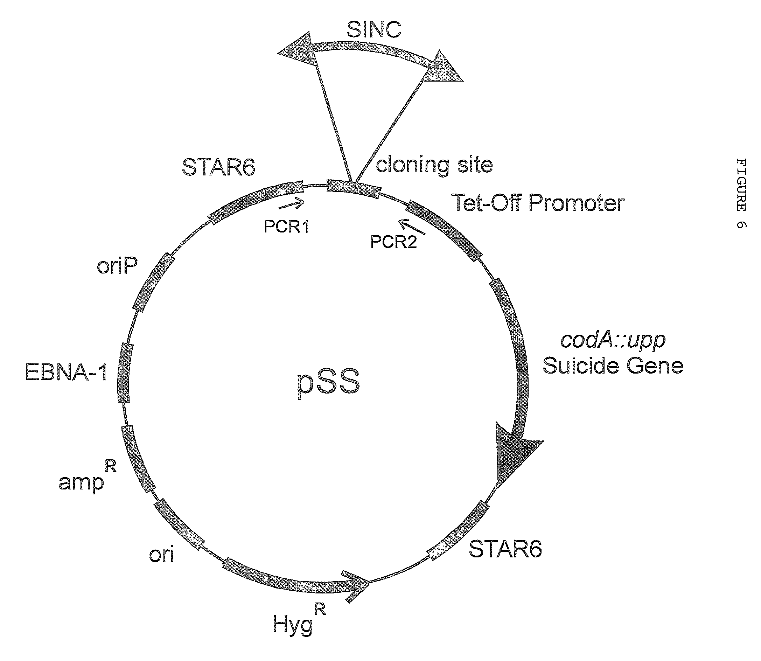

The plasmid for the SINC screen, pSINC-Select ("pSS", Figure 6) is constructed as follows: the pREP4 vector (Invitrogen V004-50) is used as the plasmid backbone. It provides the Epstein Barr oriP origin of replication and EBNA-1 nuclear antigen for high-copy episomal replication in primate cell lines; the hygromycin resistance gene with the thymidine kinase promoter and polyadenylation site, for selection in mammalian cells; and the ampicillin resistance gene and colE1 origin of replication for maintenance in *Escherichia coli.* The vector contains a Tet-Off promoter, consisting of tandem Tet Responsive Elements (TREs) from plasmid pUDH10-3 (Gossen and Bujard, 1992), for regulation by the Tet-Off transcriptional regulatory system. The TREs regulate expression of the codA::upp gene encoding a fusion protein (cyotsine deaminase/uracil phosphoribosyltransferase; Invivogen porfcodaupp). This is a so-called "suicide gene"; the activity of the codA::upp enzyme converts a pro-drug 5-fluorocytosine (5-FC) to a toxic drug, 5-fluorouracil (5-FU), thereby causing apoptosis and cell death (Mullen et al., 1992; Tiraby et al., 1998; Wei and Huber, 1996). Upstream from the Tet-Off promoter is a *Bgl*II restriction site for cloning *Sau*3AI-digested genomic DNA for screening. The pREP4 DNA is separated from the genomic DNA and suicide gene by STAR elements in order to prevent silencing of essential plasmid elements in the pREP4 component by cloned SINC elements. Genomic DNA from a library of BAC clones comprising human chromosome 22 (Invitrogen/Research Genetics 96010-22) is partially digested with *Sau*3AI and ligated into *Bgl*II-digested pSS (Sambrook et al., 1989). The library of recombinant plasmids is transfected into the U-2 OS/Tet-Off cell line by calcium phosphate precipitation (Graham and van der Eb, 1973; Wigler et al., 1978) as recommended by the supplier of the transfection reagent (Life Technologies). Transfected cells are cultured under hygromycin selection (25 µ g/ml) and tetracycline repression (doxycycline, 10 ng/ml) for 3 weeks. Then 5-FC is added to a concentration of 1 µg/ml and the cells are cultured for a further 3 weeks to select for SINC elements.

Candidate SINC-containing colonies are harvested and used in a polymerase chain reaction with primers PCR1 and PCR2 (Figure 6); the PCR products are digested with *Hin*dIII and *Xho*I restriction endonucleases and cloned into pBluescript II SK(+) (Stratagene 212207) by conventional techniques (Sambrook et al., 1989). The DNA sequences of the candidate SINC elements are determined (Sanger et al., 1977), and corresponding sequences in the human genome are identified by BLAST searches (Altschul et al., 1990) of the human genome database (http://www.ncbi.nlm.nih.gov/genome/seq/HsBlast.html 20 June 2001). The chromosomal locations of the elements are recorded, along with the proportion of repetitive DNA and the identity of adjacent genes.

### Results

At the end of the selection period no colonies are evident in the control cultures (empty pSS), and a number of colonies are evident in the cultures containing pSS with genomic DNA. These surviving clones contain candidate SINC elements. The elements are recovered by PCR and subcloned into a standard cloning vector, pBluescript. The DNA sequences of the elements are determined, and compared with the human genome sequence (Table 3). In all cases, the sequenced elements are found on chromosome 22, as expected.

### Example 2. Expression characteristics of the transgene that are due to the STAR, the SINC, or the combined STAR/SINC.

Background: site-specific recombination is used to precisely remove heterologous DNAs from their chromosomal locations. This is routinely carried out by one of two systems: the cre recombinase and loxP target of bacteriophage P1 (Feng et al., 1999), or the FLP recombinase and FRT (FLP recombinase target) of yeast (Wigley et al., 1994). In these systems, a DNA region (usually containing a reporter gene and/or a selectable marker) is flanked in the chromosome by the loxP or FRT target. The activity of the recombinase then catalyzes the precise excision of the DNA region from the chromosome. The recombinase resolves its two recognition sequences to a single site, deleting the sequence between them. Thus, a span of DNA must be flanked by target sites to be subsequently deleted in vivo upon introduction or activation of recombinase (Schwenk et al., 1995; Dymecki, 1996). The Cre and Flp recombinases catalyze recombination between two 13-base-pair inverted repeats, separated by a spacer with a minimum of 6 (loxP) or 8 (FRT) base pairs (Senecoff et al., 1985). The loxP sequence is ATAACTTCGTATA and the FRT sequence is GAAGTTCCTATAC.

Protocol: Using conventional DNA cloning (Sambrook et al., 1989), a reporter gene (encoding a reporter protein, for example green fluorescent protein (GFP) (Bierhuizen et al., 1997) or luciferase (Himes and Shannon, 2000) is constructed that is flanked in a plasmid by a pair of STAR elements, by a pair of SINC elements, or by a pair of STAR/SINC combination elements. In each case, the elements are themselves flanked by recombinase target sites. One element is flanked by a pair of loxP sites, and the other is flanked by a pair of FRT sites (Figure 1). Upon transfection the plasmid integrates into the host chromosome in a small percentage of cells, and the integrants are selected by antibiotic resistance. Similar constructs are made for each of the three test elements (STAR, SINC, STAR/SINC).

Using conventional techniques, ("SuperFect Transfection Reagent Handbook," Qiagen, November, 1997) these plasmids are transfected into the U-2 OS human osteosarcoma cell line, and selected for hygromycin resistance. Hygromycin-resistant isolates have the plasmid stably integrated in the genome of the cell line. Individual isolates are propagated in cell culture medium, and the expression of the transgenic reporter gene is assayed, for example by flow cytometry (Stull et al., 2000).

Then using conventional techniques (transfection, or hormone stimulation), the stable isolates from above are treated so as to introduce or activate recombinase activity. This is done sequentially, such that for example the cre recombinase activity catalyzes excision of STAR1, and subsequently FLP recombinase activity catalyzes excision of STAR2. The level of expression of the reporter gene in these cells is assayed and the value compared with the reference value of the parental, STAR-containing isolate.

### Example 3. Sequence analysis of STARS; determination of minimal essential sequence for element function; sequence conservation among elements; and properties of tandem and multiple elements

Background: DNA fragments containing STAR or SINC elements are isolated by genetic selection using the pSelect (Figure 1) or pSS (Figure 6) plasmids, respectively. This section describes the approach to characterize the DNA sequences within those fragments that have STAR or SINC activity. Protocols:

DNA sequence: Oligonucleotides are designed based on the sequence of the pSelect and pSS selection plasmids for sequencing the DNA fragments. The fragments are sequenced using the dideoxy chain termination technique (Sanger et al., 1977). DNA squences are then localized to chromosome position using the public human genome sequence database (http://www.ncbi.nlm.nih.gov:80/cgi-bin/Entrez/hum_srch?chr=hum_chr.inf&query). Genes and gene density in the vicinity of the fragment sequence are recorded from the genome sequence annotation. Transcriptional activity of those genes is determined from public databases of DNA microarray (http://arrays.rockefeller.edu/xenopus/links.html) and SAGE (Serial Analysis of Gene Expression; http://bioinfo.amc.uva.nl/HTM-bin/index.cgi) data.

Once positional information on STAR and SINC sequences is compiled, the data are analysed in terms of underlying consensus sequences. Consensus sequences or trends (understood by this are local areas rich in particular nucleotide combinations, e.g. rich in C and G bases) are detected using similarity search algorithms such as clustalw (Higgins et al., 1996) and blosum similarity scoring (Altschul and Gish, 1996). Any underlying consensuses or trends found are then used to identify other potential STARs on a genome scale by performing BLAST searches (Altschul et al., 1990). Previous research has identified transcriptional regulatory proteins that bind to known insulators and boundary elements (Gaszner et al., 1999; Gerasimova and Corces, 1998). In the described examples, the protein binding sites coincide with DNase I hypersensitive sites which are essential for insulator or boundary function. The hypothesis that STAR elements are also bound by known regulatory proteins is examined by searching the TRANSFAC database of transcription factors (http://transfac.gbf.de/TRANSFAC/) for sequence motifs that occur in the STAR elements. Sequence motifs that are common among the members of the STAR or SINC collections are indicators that the corresponding transcription factor binds to that element.

Minimal essential sequence: Using this sequence knowledge STAR (or SINC) elements are truncated and tested for functionality. This is done using the polymerase chain reaction (PCR) to clone sub-fragments of the STAR- or SINC-containing fragments into pSelect or pSS by standard techniques (Sambrook et al., 1989). The plasmids containing the sub-fragments are transfected into U-2 OS cells and tested for functionality by assaying for antibiotic resistance (STAR elements) or pro-drug resistance (SINC elements).

Directionality: The STAR and SINC elements are tested for their directionality using the pSelect and pSS plasmids, respectively. For example, the direction of STAR elements isolated by the pSelect screen is referred to as 5'3' orientation. The orientation of the element is reversed by conventional recombinant DNA techniques (Sambrook et al., 1989). The resulting plasmids are transfected into the U-2 OS cell line and expression of the reporter gene is assayed (Bierhuizen et al., 1997; Himes and Shannon, 2000). The level of expression from the plasmid with the reverse-orientation element is compared to that with the 5'3' orientation. If the reverse-orientation plasmid has similar expression levels, then the STAR element does not display directionality.

Combinations and multiples of elements: To determine whether STAR elements are able to function in mixed pairs, different elements are combined and tested. The analysis is performed in the pSDH plasmid by inserting one STAR element in MCSI and a different STAR in MCSII by recombinant DNA techniques (Sambrook et al., 1989). The resulting plasmids are transfected, and the expression of the reporter gene is assayed (Bierhuizen et al., 1997; Himes and Shannon, 2000); the results are compared with the expression from plasmids containing the same element at MCSI and MCSII; if the expression is similar for the two types of plasmids, then it is concluded that different STAR elements do not interfere with each other.

The strength of single STAR or SINC elements is compared with tandem repeats of elements. This is done by concatamerization of the STAR or SINC elements of interest with DNA ligase and insertion of the ligation product into the pSDH or pSS plasmids by recombinant DNA techniques (Sambrook et al., 1989). The resulting plasmids are transfected into U-2 OS cells, and the expression of the reporter gene is assayed (Bierhuizen et al., 1997; Himes and Shannon, 2000); the results are compared with the expression from plasmids containing single STAR or SINC elements.

### Example 4. Determination of the distance over which a STAR, a SINC, or a combination thereof functions.

Background: STAR elements are used to optimize expression of single and multiple transgenes. To determine if a single pair of STAR elements can protect large or multiple transgenes from silencing it is necessary to determine the range over which STAR elements act. Similar information is determined for SINC elements and STAR/SINC combinations.

Protocol: STAR and SINC elements are tested for their functionality over distance using derivative plasmids based on pSelect or pSS respectively, as follows. A library of random DNA fragments from 500bp to 10kb is assembled by standard DNA cloning techniques (Sambrook et al., 1989). Fragments are selected from this library that do not possess STAR or SINC activity, by testing in the pSelect and pSS plasmids as described above. For STAR elements and STAR/SINC combinations, these fragments are inserted between the cloning site and the promoter of the reporter gene in the appropriate pSelect plasmid (Figure 1). This set of plasmids is transfected into the U-2 OS cell line, and expression measured as described above. The strength of reporter gene expression is correlated with the length of the random DNA fragment separating the STAR element from the promoter. SINC elements are assessed in an analogous fashion: random DNA fragments are inserted between the SINC element and the promoter of the appropriate pSS plasmid, and the degree of repression of the reporter gene is correlated with the length of the random DNA fragment.

### Example 5. (a) Use of a naturally occurring SINC element in the genetic selection for STAR elements.

Background: The current screens for STAR elements use chimeric lexA-PcG proteins to provide repression of the selectable marker in the selection plasmid. By repeating the selection using naturally occurring SINC elements, STAR elements are identified that are specific to the repressive activity due to these naturally occurring SINC elements.

The SING element screen is based on the ability of genetic selection to identify randomly generated fragments of genomic DNA that are able to silence a "tet-off" promoter and block the expression of the codA::upp suicide gene. The SINC elements recovered from this selection represent a random sampling of genomic silencing elements, and different classes of elements are recovered. For this protocol, these diverse SINC elements are used to recover different classes of STAR elements than those recovered in the aforementioned lexA-PcG based selections.

Protocol: SINC elements from the current selection are characterized and sorted into classes on the basis of of functional and DNA sequence features (functional features include strength of repression; sequence features include identifiable conserved motifs; see example 3). Representative elements from each class are used to replace the lexA binding sites in the pSelect plasmid via standard DNA cloning techniques (Sambrook et al., 1989). A gene bank is made with each of these new plasmids, and used to identify new, SINC-specific STAR elements as described (van der Vlag et al., 2000). This is done with whole genomic DNA, and with DNA from the BAC clone that also contains the SINC element being used.

### Example 5 (b) Determination of the maximal length of STAR and SINC elements

Background: STAR elements are cloned as fragments of DNA recovered using the pSelect plasmid, which is done with genomic DNA fragments less than 2 kb. However, these might be portions of a more extended STAR element. Extended STAR activity is examined by the following experiments.

Protocol: STAR elements cloned in pSelect are mapped to the human genome sequence. In order to determine if they are portions of a more extended STAR element, regions of 4 kb that encompass the clones are amplified by PCR and cloned into the pSelect and/or pSDH plasmid by standard recombinant DNA techniques (Sambrook et al., 1989). The resulting plasmids are transfected into U-2 OS cells and assayed for reporter gene expression as described above; plasmids containing the original 2 kb STAR element are included as a control.

Three possible results can be expected: (1) similar expression by the control and extended STAR isolates, demonstrating that the STAR element is confined to the original 2 kb fragment; (2) lower expression by the extended STAR isolates, suggesting that the STAR element is contained within the 2 kb fragment and does not act effectively over a distance or that the extended fragment contains a SINC element; (3) higher expression by the extended STAR isolates, suggesting that the extended region contains a more complete STAR element. In the case of result (3), the exercise is reiterated with a larger PCR fragment of 6 kb.

A STAR element may also be a composite of sites to which various proteins bind. Therefore large DNA fragments with STAR activity could be divisible into smaller fragments with STAR activity (see example 3). Elements that are greater than 2 kb are recognized as STAR elements if they still display STAR activity after truncation to less than 2 kb (including by internal deletion).

### Example 6. Methylation and histone acetylation states of STAR elements, SINC elements, or combinations thereof and of the adjacent transgenes.

Background: The regulatory properties of STAR and SINC elements are associated with the local chromatin structure, which is determined by the DNA itself and by DNA-associated proteins. Changes in chromatin structure that are associated with changes in gene expression are often produced by secondary modifications of the macromolecules, especially methylation of DNA or acetylation of histone proteins. Identifying the secondary modifications occurring at STAR and SINC elements and at adjacent transgenes provides hallmarks for these elements.

Protocol: DNA methylation: STAR or SINC elements or combinations thereof are cloned into the pSelect plasmid by standard techniques (Sambrook et al., 1989). U-2 OS cells are stably transfected with these plasmids, and with pSelect lacking a STAR or SINC element as a control to determine basal DNA methylation at the reporter gene. Cells are harvested and the chromatin purified by standard procedures (Thomas, 1998). The DNA is digested with the *Hpa*II and *Msp*I restriction endonucleases in separate reactions (Sambrook et al., 1989). Both of these restriction enzymes are able to cut the non-methylated sequence CCGG. When the external C is methylated, both *Msp*I and *Hpa*II cannot cleave. However, unlike *Hpa*II*, Msp*I can cleave the sequence when the internal C is methylated. The DNA is subjected to Southern blotting and the blot is analyzed by indirect end-labeling (Pazin and Kadonaga, 1998). As a control, the corresponding pSelect plasmid as naked, unmethylated DNA, is also cut with the described enzymes and subjected to Southern blotting. Comparison of the different sizes of the DNA fragments reveals whether the DNA is methylated in vivo or not.

Histone acetylation: The same transfected cell lines used for DNA methylation analysis are used for these experiments. The method described below yields a high resolution map of the histone acetylation pattern on the STAR and SINC elements and the reporter gene (Litt et al., 2001). Micrococcal nuclease digests of nuclei are fractionated on sucrose gradients, and purified nucleosome monomers and dimers are enriched for acetylated histones by immunoprecipitation with anti-acetylhistone antibodies. The nucleosome fraction and immunoprecipitates are subjected to analysis, for example by real-time PCR (Jung et al., 2000) using primers and a Taqman probe that anneal to the reporter gene or to the STAR or SINC element to yield 0.2 kb products, with a moving window of 0.1 kb. The rate of increase of the Taqman probe fluorescent signal during the PCR (which is proportional to the abundance of the template DNA in the sample) is then measured. The ratio of the abundance of the template DNA in the nucleosome fraction and the immunoprecipitates provides a fine-map of the pattern of histone acetylation for each 0.1 kb on the reporter gene and STAR or SINC element (or on the reporter gene in the absence of an element).

### Example 7. In vivo Nucleosome positioning and DNAse I hypersensitive sites

Background: Chromatin is comprised of DNA, histones, and non-histone proteins. The histones form a core particle that is wrapped by ∼150 bp of DNA to make a nucleosome. Nucleosomes are separated by 50-75 bp of linker DNA. Stably positioned nucleosomes on chromosomal DNA repress gene expression, and factors that exclude nucleosomes or otherwise remodel chromatin can overcome this repression. The positioning of nucleosomes in a chromosomal region is analyzed by micrococcal nuclease (MNase) assay; MNase cuts chromatin preferentially in the linker DNA. Similarly, some areas of DNA are constitutively exposed to non-histone proteins, and these are frequently regulatory regions, i.e. sites where cis-acting regulatory factors bind. Experimentally, these site are hypersensitive to digestion by the enzyme DNase I.

Protocol: To determine the position of nucleosomes on the reporter gene and on either the STAR or SINC elements, MNase is used (Saluz and Jost, 1993). Nuclei are purified from cultured U-2 OS cells and digested with MNase as described above (histone acetylation). To search for DNase I hypersensitive sites in the STAR and SINC elements or the reporter gene, purified nuclei are treated with DNase I at an appropriate concentration (e.g. 100 µg/ml genomic DNA and 20-100 U/ml DNaseI), as described (Wallrath et al., 1998). Naked DNA is digested with DNase I as a control. For both techniques, the reporter gene and STAR or SINC elements are fine-mapped using primer extension or indirect end-labelling and Southern blotting, as described (Tanaka et al., 1996; van der Vlag et al., 2000). The MNase assay reveals a ladder of discrete bands on an autoradiogram corresponding to the positions of nucleosomes on the STAR or SINC elements or the reporter gene. DNase I hypersensitive sites are manifested as discrete bands in the resulting autoradiogram that are absent or less prominent in the naked DNA control.

### Example 8. Cell-type, tissue dependence, and promoter dependence of STAR and SINC elements.

Background: It has been reported that some insulators or boundary elements may display tissue specificity (Takada et al., 2000). STAR elements have many features in common with insulators and boundary elements. Both promiscuous and tissue-specific STAR and SINC elements have biotechnological value in transgenic applications. The assay described below is performed to assess cell-type dependence. Cell and tissue specificity of the elements are examined further by examining the expression of genes in the vicinity of the elements in the human genome, using public databases of DNA microarray (http://arrays.rockefeller.edu/xenopus/links.html) and SAGE (Serial Analysis of Gene Expression; http://bioinfo.amc.uva.nl/HTM-bin/index.cgi) data.

Protocol: STAR elements are tested in the pSDH plasmid, and SINC elements in the pSS plasmid. Three cell lines are transfected using standard protocols: the human U-2 OS osteosarcoma cell line (Heldin et al., 1986), the Vero cell line from African green monkey kidney (Simizu et al., 1967), and the CHO cell line from Chinese hamster ovary (Kao and Puck, 1968). Elements able to function in all three cell types are catagorized as promiscuous. Those only displaying activity in one or two of the cell-lines are catagorized as restricted in their cell-type functionality.

Promoter specficity: STAR and SINC elements are currently selected and tested in the context of function with two promoters, the entire cytomegalovirus (CMV) promoter or the Tetracycline Response Element and minimal CMV promoter (in combination with the tTA transcriptional activator). To assess promoter specificity, STAR and SINC function are tested with other commonly used viral promoters, namely the simian virus type 40 (SV40) early and late promoters, the adenoviral E1A and major late promoters, and the Rous sarcoma virus (RSV) long terminal repeat (Doll et al., 1996; Smith et al., 2000; Weaver and Kadan, 2000; Xu et al., 1995). Each of these promoters are cloned separately into the pSelect and pSS plasmids by standard techniques (Sambrook et al., 1989) along with STAR or SINC elements, respectively. The resulting plasmids are transfected into the U-2 OS cell line and assayed for reporter gene expression, as described above. The ability of SINC elements to silence these promoters, or STAR elements to protect against silencing, is determined by comparison with plasmids lacking STAR or SINC elements.

### Example 9. Methods for improvement of STAR and SINC elements

Background: Improved STAR and SINC elements are developed. Improvements yield increased strength of anti-repressive or repressive activity, and elements with inducible and tissue-specific activity. These improvements are made by a combination of techniques.

### Protocols

Forced evolution: Error prone PCR (Cherry et al., 1999; Henke and Bornscheuer, 1999) is used to introduce an average of one to two point mutations per element. The mutagenized elements are screened using pSelect (or pSS) plasmids containing reporter-selectable marker fusion proteins by for example fluorescence activated cell sorting and antibiotic resistance (Bennett et al., 1998). Subsequent rounds of error prone PCR and selection are carried out to derive elements with further improvements in activity.

Tandem and heterologous combinations: As described above, tandem and heterologous combinations of elements are tested for activity in comparison with the single elements (example 3).

The relative dominance of STAR and SINC elements is tested on a case by case basis. It is used to test the strength of an element; for example, if a new STAR element is dominant to a known, strong SINC element, then the STAR is classified as very strong. The possibility that the dominance relationship between a STAR and a SINC is cell type-, tissue-, or promoter-specific is also considered (example 8). The dominance test utilizes the pSelect plasmid, with individual SINC elements placed upstream of individual STAR elements by standard recombinant DNA techniques (Sambrook et al., 1989). The plasmids are transfected to U-2 OS cells and reporter gene expression is assayed. SINC dominance is manifested by lower expression than the plasmid with only a STAR element, while STAR dominance is manifested by higher expression than the plasmid with only a SINC element.

Introduction of binding sites for other DNA-binding proteins to STAR and SINC elements to add novel characteristics (e.g. inducibility, tissue specificity) Background: Regulatable STAR and SINC elements are created by combining them with binding sites for signal-dependent DNA binding proteins. In one example this would involve juxtaposition of a STAR or SINC or STAR/SINC combination and a glucocorticoid response element (GRE). In the absence of glucocorticoid stimulation the STAR or SINC element would function as described. Upon stimulation, the naturally occurring glucocorticoid receptor binds to the GRE and interferes with STAR or SINC function.

Protocol: Using conventional DNA cloning (Sambrook et al., 1989), a GRE is introduced into the pSelect or pSS vector adjacent to STAR or SINC elements, respectively. The plasmid is transfected into U-2 OS cells as described above. Cells are divided into two cultures; one is treated with glucocorticoid (10 µM). Expression of the reporter gene is measured and compared between the two cultures. Differences in expression demonstrate the ability to regulate STAR and SINC function by action of a signal-dependent DNA-binding protein.

Promiscuous STAR and SINC elements: Testing or enhancing these characteristics involves cultivation in different cell lines, and long term cultivation without antibiotic selection (examples 8 and 10).

### Example 10. STAR and SINC elements obviate the need for continuous selection for maintenance of the transgene.

Background: In transgenesis, reliance on selection markers has two drawbacks: the selection agent is usually expensive and carries a metabolic cost to the cells, and there are regulatory and ethical objections to including selectable markers in transgenic applications, especially if the transgene itself is in the product (e.g. crop plants, gene therapy vectors). STAR and SINC elements reduce or eliminate the need to maintain selection after establishing the transgenic isolate. Consequently, the resistance gene can be removed from the transgenic genome by site-specific recombination with diminished loss of transgene expression.

Protocol: Stably transfected U-2 OS cell lines containing chromosomally-integrated STAR elements flanking reporter genes are produced by co-transfection of the pSDH plasmid with a trans-acting antibiotic resistance plasmid as described above. The experiment involves testing the stability of the reporter gene expression level in these cell lines during prolonged (3-6 month) cultivation in the absence of selection. This is tested with STAR elements flanking the luciferase or GFP reporter genes in pSDH plasmids. The antibiotic resistance gene is removed by constructing an expression plasmid (based on pSDH) in which the antibiotic selection marker is flanked by recombinase target sites. The selectable marker is subsequently excised by recombinase activity, as described above (example 2).

### Brief description of the drawings

Figure 1. The pSelect family of plasmids for selecting and characterizing STAR elements. A resistance marker (zeocin or puromycin) or reporter gene (GFP or luciferase) under control of the promiscuous SV40 promoter is adjacent to a BamHI cloning site flanked by AscI and HindIII sites. Upstream of the cloning site are lexA operators to which lexA protein can bind. Binding of chimeric lexA-Polycomb group proteins to the operators causes repression of the marker or reporter gene. DNA fragments inserted at the cloning site that block repression are identified by the persistent expression of the marker or reporter gene. The plasmid replicates episomally in cultured mammalian cells due to the oriP seqeunce.

Figure 2. The pSDH family of plasmids for testing STAR elements. Two multiple cloning sites (MCSI and MCSII) flank a reporter gene (GFP or luciferase) whose expression is driven by an upstream promoter (CMV, Tet-off, or SV40). STAR elements to be tested are inserted at MCSI and MCSII. These contain unique restriction sites (MCSI: XhoI, NotI, EcoRI, and SalI; MCSII, HindIII, EcoRV, BglII, and NheI). The plasmid replicates after integrating at random in the genome of mammalian cells.

Figure 3. Proportion of clones over-expressing luciferase. U-2 OS human osteosarcoma cells were stably transfected with pSDH plasmids (containing the luciferase reporter gene under control of the tet-off promoter), and individual transfected clones were isolated and cultivated. Luciferase expression was measured enzymatically. The average luciferase expression by clones containing the STARless pSDH ("reference level") was determined. Clones from the sets for all plasmids were scored as "over-expressing" if their luciferase activity was more than 2-fold higher than the reference level. The percentage of over-expressing clones in each plasmid set in plotted.

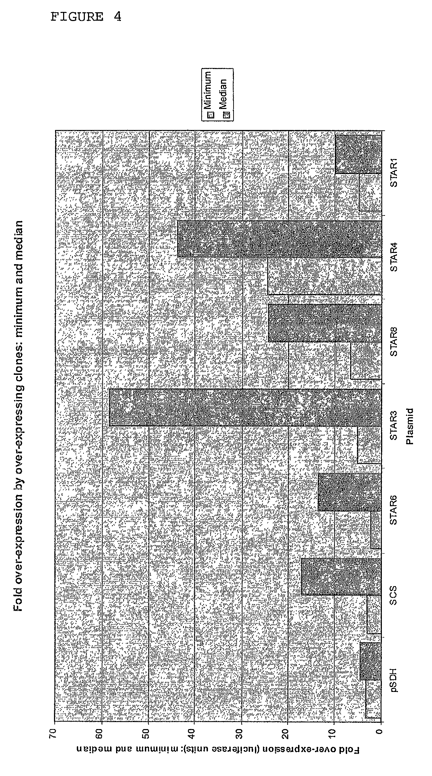

Figure 4. Fold over-expression by over-expressing clones. The range of over-expression in STAR-containing pSDH plasmids integrated into genomic DNA was determined by dividing the luciferase activities of each clone by the reference level. For those displaying significant expression (more than 2-fold above the reference level), the actual fold increases were noted; the minimum and median values of these data are plotted for each plasmid.

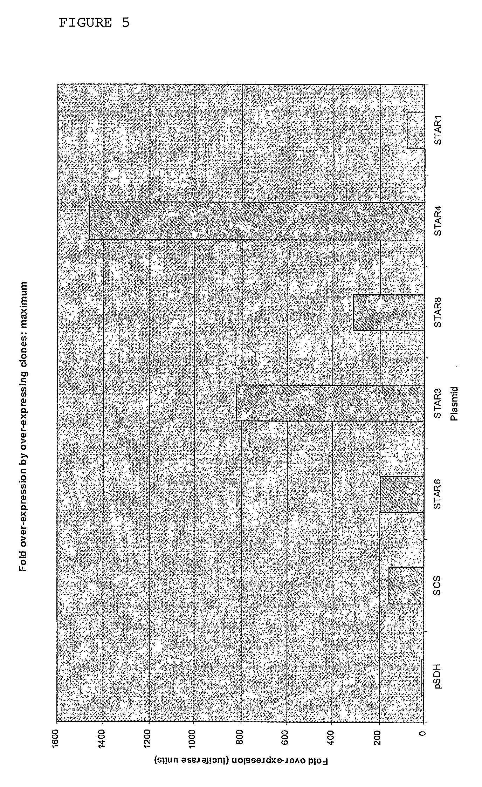

Figure 5. Fold over-expression by over-expressing clones. The range of over-expression in STAR-containing pSDH plasmids integrated into genomic DNA was determined by dividing the luciferase activities of each clone by the reference level. For those displaying significant expression (more than 2-fold above the reference level), the actual fold increases were noted; the maximum values of these data are plotted for each plasmid.

Figure 6. The pSS (SINC-Select) plasmid for selecting and characterizing SINC elements. The codA::upp suicide gene encodes a protein that converts the pro-drug 5-fluorocytosine to the toxic drug 5-fluorouracil. Upon induction by lowered tetracycline concentration, host cells become sensitive to the pro-drug. Genomic DNA fragments inserted at the cloning site (BglII-XhoI) that have silencing activity will prevent expression of the suicide gene and allow formation of pro-drug resistant colonies. STAR elements flank the selection components to prevent spreading of silenced chromatin to the functional components of the plasmid. The plasmid replicates episomally in cultured mammalian cells due to the oriP seqeunce.

### References

Altschul, S.F. and Gish, W. (1996) Local alignment statistics. Methods Enzymol, 266, 460-480.
Altschul, S.F., Gish, W., Miller, W., Myers, E.W. and Lipman, D.J. (1990) Basic local alignment search tool. J Mol Biol, 215, 403-410.
Bennett, R.P., Cox, C.A. and Hoeffler, J.P. (1998) Fusion of green fluorescent protein with the Zeocin-resistance marker allows visual screening and drug selection of transfected eukaryotic cells. Biotechniques, 24, 478-482.
Bierhuizen, M.F., Westerman, Y., Visser, T.P., Wognum, A.W. and Wagemaker, G. (1997) Green fluorescent protein variants as markers of retroviral-mediated gene transfer in primary hematopoietic cells and cell lines. Biochem Biophys Res Commun, 234, 371-375.
Breckenridge, D.G. and Shore, G.C. (2000) Regulation of apoptosis by E1A and Myc oncoproteins. Crit Rev Eukaryot Gene Expr 10, 273-280.
Bunker, C.A. and Kingston, R.E. (1994) Transcriptional repression by Drosophila and mammalian Polycomb group proteins in transfected mammalian cells. Mol Cell Biol, 14, 1721-1732.
Cherry, J.R., Lamsa, M.H., Schneider, P., Vind, J., Svendsen, A., Jones, A. and Pedersen, A.H. (1999) Directed evolution of a fungal peroxidase. Nat Biotechnol, 17, 379-384.
Doll, R.F., Crandall, J.E., Dyer, C.A., Aucoin, J.M. and Smith, F.I. (1996) Comparison of promoter strengths on gene delivery into mammalian brain cells using AAV vectors. Gene Ther, 3, 437-447.
Eissenberg, J.C., James T.C., Foster-Hartnett D.M., Hartnett T., Ngan V., and Elgin S.C.R. (1990) Mutation in a heterochromatin-specific chromosomal protein is associated with suppression of position-effect variegation in *Drosophila melanogaster.* Proc Natl Acad Sci (USA) 87: 9923-9927.
Feng, Y.Q., Seibler, J., Alami, R., Eisen, A., Westerman, K.A., Leboulch, P., Fiering, S. and Bouhassira, E.E. (1999) Site-specific chromosomal integration in mammalian cells: highly efficient CRE recombinase-mediated cassette exchange. J Mol Biol, 292, 779-785.
Gaszner, M., Vazquez, J. and Schedl, P. (1999) The Zw5 protein, a component of the scs chromatin domain boundary, is able to block enhancer-promoter interaction. Genes Dev, 13, 2098-2107.
Gerasimova, T.I. and Corces, V.G. (1998) Polycomb and trithorax group proteins mediate the function of a chromatin insulator. Cell, 92, 511-521.
Gossen, M. and Bujard, H. (1992) Tight control of gene expression in mammalian cells by tetracycline-responsive promoters. Proc Natl Acad Sci U S A, 89, 5547-5551.
Graham, F.L. and van der Eb, A.J. (1973) Transformation of rat cells by DNA of human adenovirus 5. Virology, 54, 536-539.
Heldin, C.H., Johnsson, A., Wennergren, S., Wernstedt, C., Betsholtz, C. and Westermark, B. (1986) A human osteosarcoma cell line secretes a growth factor structurally related to a homodimer of PDGF A-chains. Nature, 319, 511-514.
Henke, E. and Bornscheuer, U.T. (1999) Directed evolution of an esterase from Pseudomonas fluorescens. Random mutagenesis by error-prone PCR or a mutator strain and identification of mutants showing enhanced enantioselectivity by a resorufin-based fluorescence assay. Biol Chem, 380, 1029-1033.
Higgins, D.G., Thompson, J.D. and Gibson, T.J. (1996) Using CLUSTAL for multiple sequence alignments. Methods Enzymol, 266, 383-402.
Himes, S.R. and Shannon, M.F. (2000) Assays for transcriptional activity based on the luciferase reporter gene. Methods Mol Biol, 130, 165-174.
Jung, R., Soondrum, K. and Neumaier, M. (2000) Quantitative PCR. Clin Chem Lab Med, 38, 833-836.
Kao, F.T. and Puck, T.T. (1968) Genetics of somatic mammalian cells, VII. Induction and isolation of nutritional mutants in Chinese hamster cells. Proc Natl Acad Sci U S A, 60, 1275-81.
Kellum, R. and Schedl, P. (1992) A group of scs elements function as domain boundaries in an enhancer-blocking assay. Mol Cell Biol, 12, 2424-2431.
Litt, M.D., Simpson, M., Recillas-Targa, F., Prioleau, M.N. and Felsenfeld, G. (2001) Transitions in histone acetylation reveal boundaries of three separately regulated neighboring loci. EMBO J, 20, 2224-2235.
Morgenstern, J.P. and Land, H. (1990) Advanced mammalian gene transfer: high titre retroviral vectors with multiple drug selection markers and a complementary helper-free packaging cell line. Nucleic Acids Res, 18, 3587-3596.
Mullen, C.A., Kilstrup, M. and Blaese, R.M. (1992) Transfer of the bacterial gene for cytosine deaminase to mammalian cells confers lethal sensitivity to 5-fluorocytosine: a negative selection system. Proc Natl Acad Sci U S A, 89, 33-37.
Nan, X., Javier Campoy, F., and Bird A. (1997) MeCP2 is a transcriptional repressor with abundant binding sites in genomic chromatin. Cell 88, 471-481.
Pazin, M.J. and Kadonaga, J.T. (1998) Transcriptional and structural analysis of chromatin assembled in vitro. In Gould, H. (ed.) Chromatin: A Practical Approach. Oxford University Press, Oxford, pp. 172-194.
Pietersen, A. and H.M. Noteborn. (2000) Apoptin. Adv Exp Med Biol 465, 153-161.
Saluz, H.P. and Jost, J.P. (1993) Approaches to characterize protein-DNA interactions in vivo. Crit Rev Eukaryot Gene Expr, 3, 1-29.
Sambrook, J., Fritsch, E.F. and Maniatis, T. (1989) Molecular Cloning: A Laboratory Manual. Cold Spring Harbor Laboratory Press, Plainview NY.
Sanger, F., Nicklen, S. and Coulson, A.R. (1977) DNA sequencing with chain-terminating inhibitors. Proc Natl Acad Sci U S A, 74, 5463-5467.
Simizu, B., Rhim, J.S. and Wiebenga, N.H. (1967) Characterization of the Tacaribe group of arboviruses. I. Propagation and plaque assay of Tacaribe virus in a line of African green monkey kidney cells (Vero). Proc Soc Exp Biol Med, 125, 119-123.
Smith, R.L., Traul, D.L., Schaack, J., Clayton, G.H., Staley, K.J. and Wilcox, C.L. (2000) Characterization of promoter function and cell-type-specific expression from viral vectors in the nervous system. J Virol, 74, 11254-11261.
Stull, R.A., Hyun, W.C. and Pallavicini, M.G. (2000) Simultaneous flow cytometric analyses of enhanced green and yellow fluorescent proteins and cell surface antigens in doubly transduced immature hematopoietic cell populations. Cytometry, 40, 126-134.
Takada, T., Iida, K., Akasaka, K., Yasue, H., Torii, R., Tsujimoto, G., Taira, M. and Kimura, H. (2000) Evaluation of heterologous insulator function with regard to chromosomal position effect in the mouse blastocyst and fetus. Mol Reprod Dev, 57, 232-237.
Tanaka, S., Livingstone-Zatchej, M. and Thoma, F. (1996) Chromatin structure of the yeast URA3 gene at high resolution provides insight into structure and positioning of nucleosomes in the chromosomal context. J Mol Biol, 257, 919-934.
Thomas, J.O. (1998) Isolation and fractionation of chromatin and linker histones. In Gould, H. (ed.) Chromatin: A Practical Approach. Oxford University Press, Oxford, pp. 1-34.
Tiraby, M., Cazaux, C., Baron, M., Drocourt, D., Reynes, J.P. and Tiraby, G. (1998) Concomitant expression of E. coli cytosine deaminase and uracil phosphoribosyltransferase improves the cytotoxicity of 5-fluorocytosine. FEMS Microbiol Lett, 167, 41-49.
van der Vlag, J., den Blaauwen, J.L., Sewalt, R.G., van Driel, R. and Otte, A.P. (2000) Transcriptional repression mediated by polycomb group proteins and other chromatin-associated repressors is selectively blocked by insulators. J Biol Chem, 275, 697-704.
Wallrath, L.L., Swede, M.J. and Elgin, S.C.R. (1998) Mapping chromatin structure in Drosophila. In Gould, H. (ed.) Chromatin: A Practical Approach. Oxford University Press, Oxford, pp. 59-77.
Weaver, L.S. and Kadan, M.J. (2000) Evaluation of adenoviral vectors by flow cytometry. Methods, 21, 297-312.
Wei, K. and Huber, B.E. (1996) Cytosine deaminase gene as a positive selection marker. J Biol Chem 271, 3812-3816.
Wigler, M, Pellicer, A., Silverstein, S. and Axel, R. (1978). Biochemical transfer of single-copy eukaryotic genes using total cellular DNA as donor. Cell 14, 725-731.
Wigley, P., Becker, C., Beltrame, J., Blake, T., Crocker, L., Harrison, S., Lyons, I., McKenzie, Z., Tearle, R., Crawford, R. and et al. (1994) Site-specific transgene insertion: an approach. Reprod Fertil Dev, 6, 585-588.
Xu, Z.Z., Krougliak, V., Prevec, L., Graham, F.L. and Both, G.W. (1995) Investigation of promoter function in human and animal cells infected with human recombinant adenovirus expressing rotavirus antigen VP7sc. J Gen Virol, 76, 1971-1980.
Yin, D.X., Zhu, L., and Schimke R.T. (1996) Tetracyclin-controlled gene expression system achieves high-level and quantitative control of gene expression. Analyt Biochem 235, 195-201.

**Table 1.**

| STAR elements improve transgene expression. | | | |
|---|---|---|---|
| Plasmid | Over-expressing clones, % | Fold over-expression (range) | Number of clones |
| Empty | 12 | 3-11 | 25 |
| SCS (positive control) | 24 | 3-160 | 21 |
| STAR-6 | 62 | 2-200 | 26 |
| STAR-3 | 39 | 5-820 | 23 |
| STAR-8 | 63 | 7-315 | 19 |
| STAR-4 | 31 | 25-1500 | 13 |
| STAR-1 | 57 | 5-80 | 23 |

Expression of the luciferase reporter gene is measured in cell lines containing integrated pSDH plasmids, without ("empty," the negative control) or containing STAR elements (including the positivie control element, SCS from *Drosophila*). The mean expression level of the negative control is defined as the reference level, and clones are considered over-expressing if their expression level is >2-fold above the reference level. The percentage of over-expressing clones for each plasmid and the fold over-expression is reported, along with the number of clones analyzed for each plasmid.

**Table 2.**

| Cloned STAR element. | | | |
|---|---|---|---|
| Clone | Chromosomal location¹ | Adjacent genes² | Repeat sequence |
| STAR-1 | N.d. | | |
| STAR-2 | N.d. | | |
| STAR-3 | For 5q33.3 Rev 10q22.2 | Chr10 part in Histone. Acetyltransferase gene | |
| STAR-4 | For 1p31.1 Rev 14q24.1 | No genes within 10 kb Intron of Regulator of G-protein signalling | 83% repetitive LINE2 & LTR ERV_Class1 |
| STAR-5 | For 3q13.1 Rev 10q22.1* | | |
| STAR-6 | 2p21 | L5 kb Unknown putative kinase R 20 kb Microtuble associated protein | 19% SINE (MIR) 29% LINE |
| STAR-7 | 1q32.2 | | 12% Alu 4% MIR (SINE) LINE1 2.5% L31CR1 11.5% MER1 7% Low complex 2% |
| STAR-8 | 9q32 | ZFP KRAB box containing Zinc Finger Protein | 35% ERV_ClassI (LTR) 2% simple repeat |
| STAR-9 | See STAR4 | | |
| STAR-10 | N.d. | | |
| STAR-11 | 2p25.1 | R 15 kb unknown DNA binding protein inhibitor (Myc type) | 12% Alu (SINE) 26% MalRs (LINE) |
| STAR-12 | 5q35.3 | R 15 kb unknown ADAM TS2 family metallo proteinase | 3% Low complexity |
| STAR-13 | See STAR4 and 9 | | |
| STAR-14 | F N.d. R 20q13.33 | | |
| STAR-15 | 1p36.36 | L 6 kb Voltage-gated K channel subunit R 4 kb unknown | 14% LTR (MaLRs) |
| STAR-16 | F 8p23.1 R 8p22 etc. | | No repeat on sequenced parts |
| STAR-17 | 2q31.1 | L 6 kb BTEB1 transcription factor R 40 kb HNRNP | 10% simple and low complexity |

| | | | |
|---|---|---|---|
| ¹Chromosomal location is determined by BLAST search of DNA sequence data from the STAR clones against the human genome database. The location is given according to standard nomenclature referring to the cytogenetic ideogram of each chromosome; e.g. 1p2.3 is the third cytogenetic sub-band of the second cytogenetic band of the short arm of chromosome 1 (http://www.ncbi.nlm.nih.gov/Class/MLACourse/Genetics/chrombanding.html). F, forward sequencing reaction result; R, reverse sequencing reaction result.N.d., not yet determined. | | | |
| ²Based on Human Genome Map View Build 22 (http://www.ncbi.nlm.nih.gov/cgibin/Entrez/hum_srch?chr=hum_chr.inf&query April 2001). L, left; R, right. | | | |
| *Position ambiguous, several hits | | | |

**Table 3.**

| SINC elements recovered from human chromosome 22 by selection in the pSS vector. | | | |
|---|---|---|---|
| SINC | Length (nt) | Chromosomal location¹ | Remarks |
| psinks 9 | 700 | 22q11.21 | Contains LTR; nearest gene ZNF 74, an RNA binding protein. LTR very repetitive. |
| psinks 12 | 750 | 22q12.3 | Located in intron of acetylglucosaminyl-transferase-like protein (664 kb) implicated in tumour formation. |
| psinks 19 | 600 | 22q13.1 | Located in intron of calcium channel, almost exclusively expressed in brain. |
| psinks 28 | 950 | 22q13.31 | Located in intron of kidney protein of unknown function. Contains SINEelement |
| psinks 30 | 700 | 22q13.33 | Contains part of SINE. |
| psinks 35 | 650 | 22q11.21 | Covers exon for solute carrier. (Nuclear gene for mitochondrion). |

| | | | |
|---|---|---|---|
| ¹Chromosomal location is determined by BLAST search of DNA sequence data from the STAR clones against the human genome database. The location is given according to standard nomenclature referring to the cytogenetic ideogram of each chromosome; e.g. 1p2.3 is the third cytogenetic sub-band of the second cytogenetic band of the short arm of chromosome 1 (http://www.ncbi.nlm.nih.gov/Class/MLACourse/Genetics/chrombanding.html). | | | |

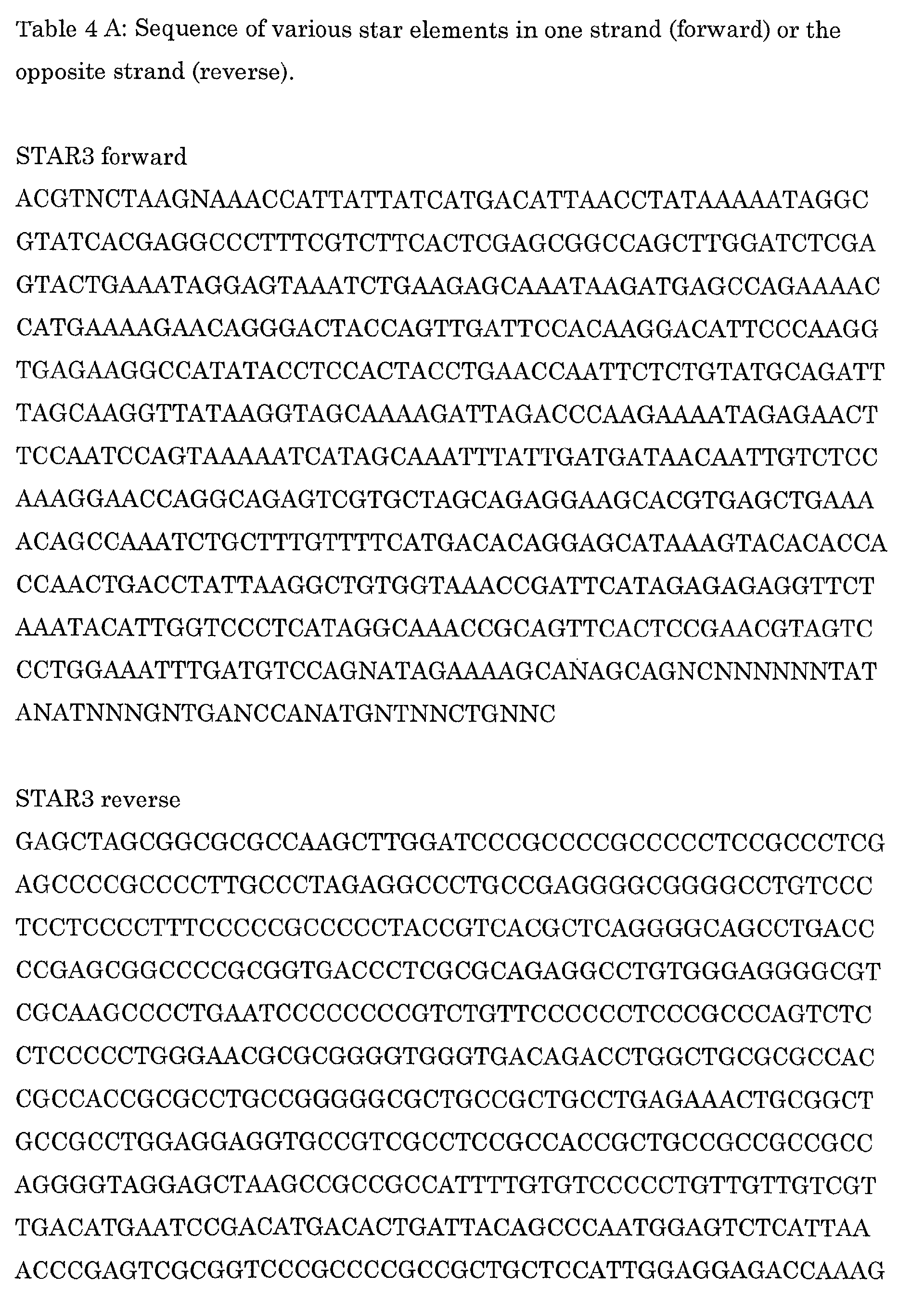

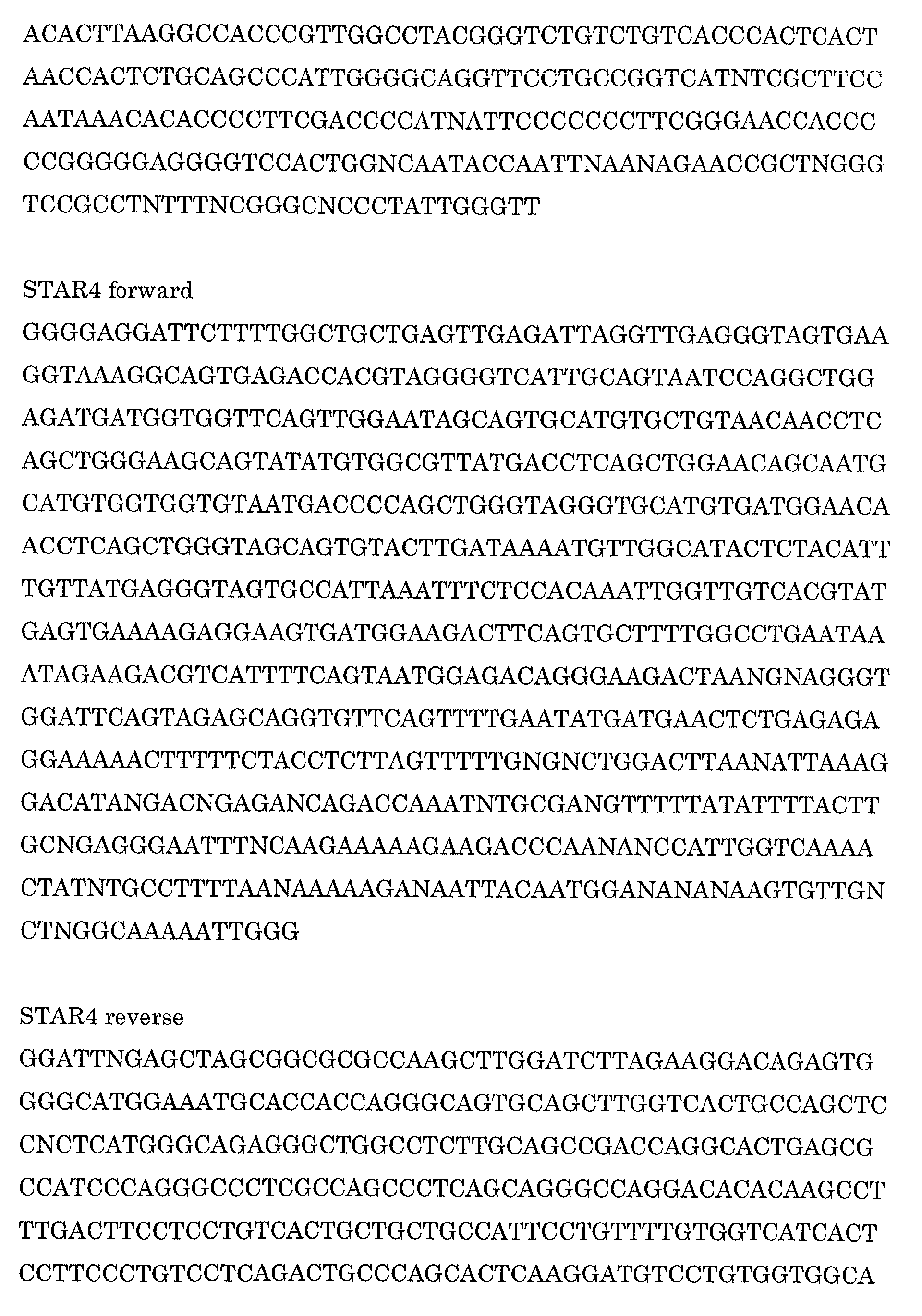

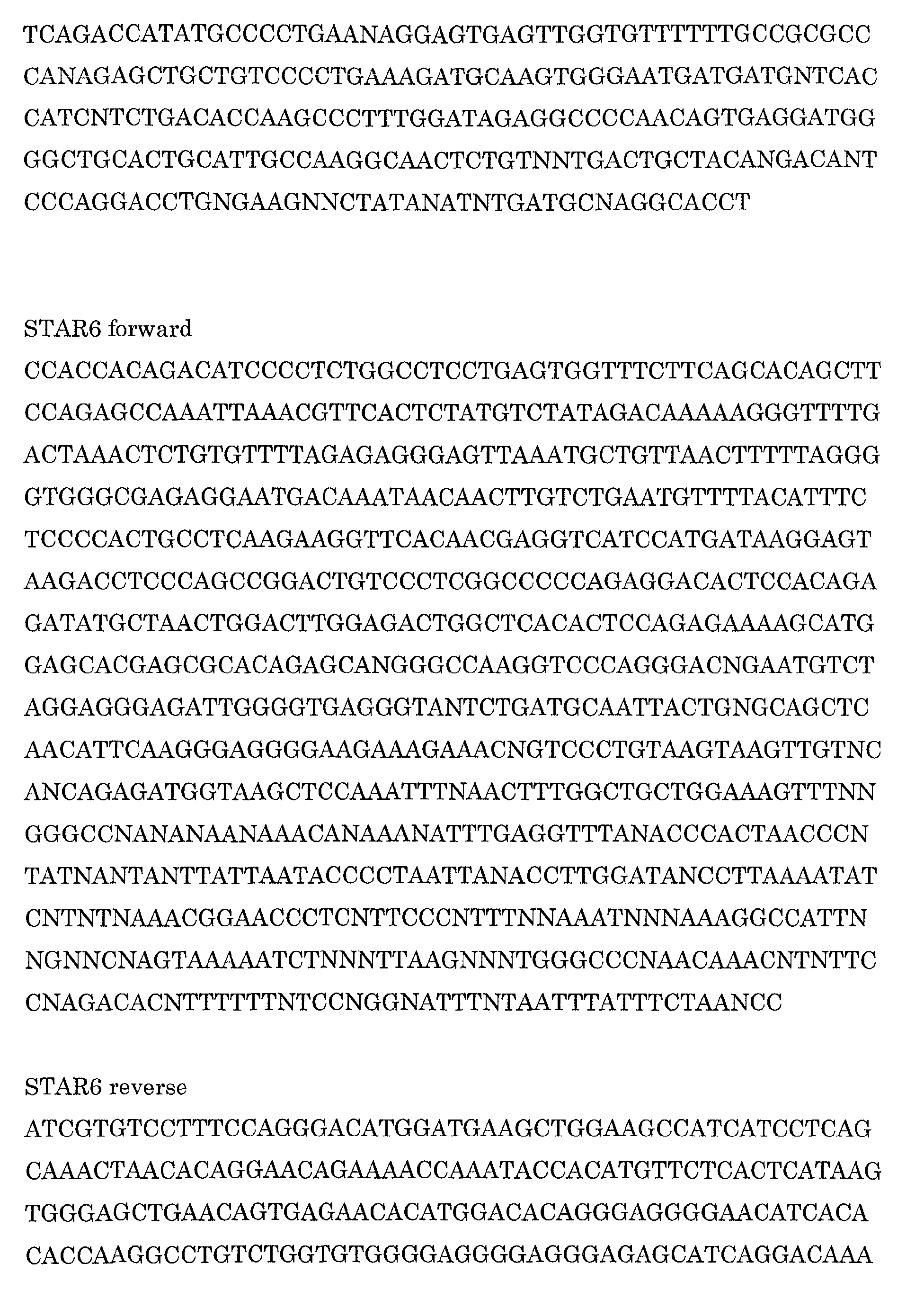

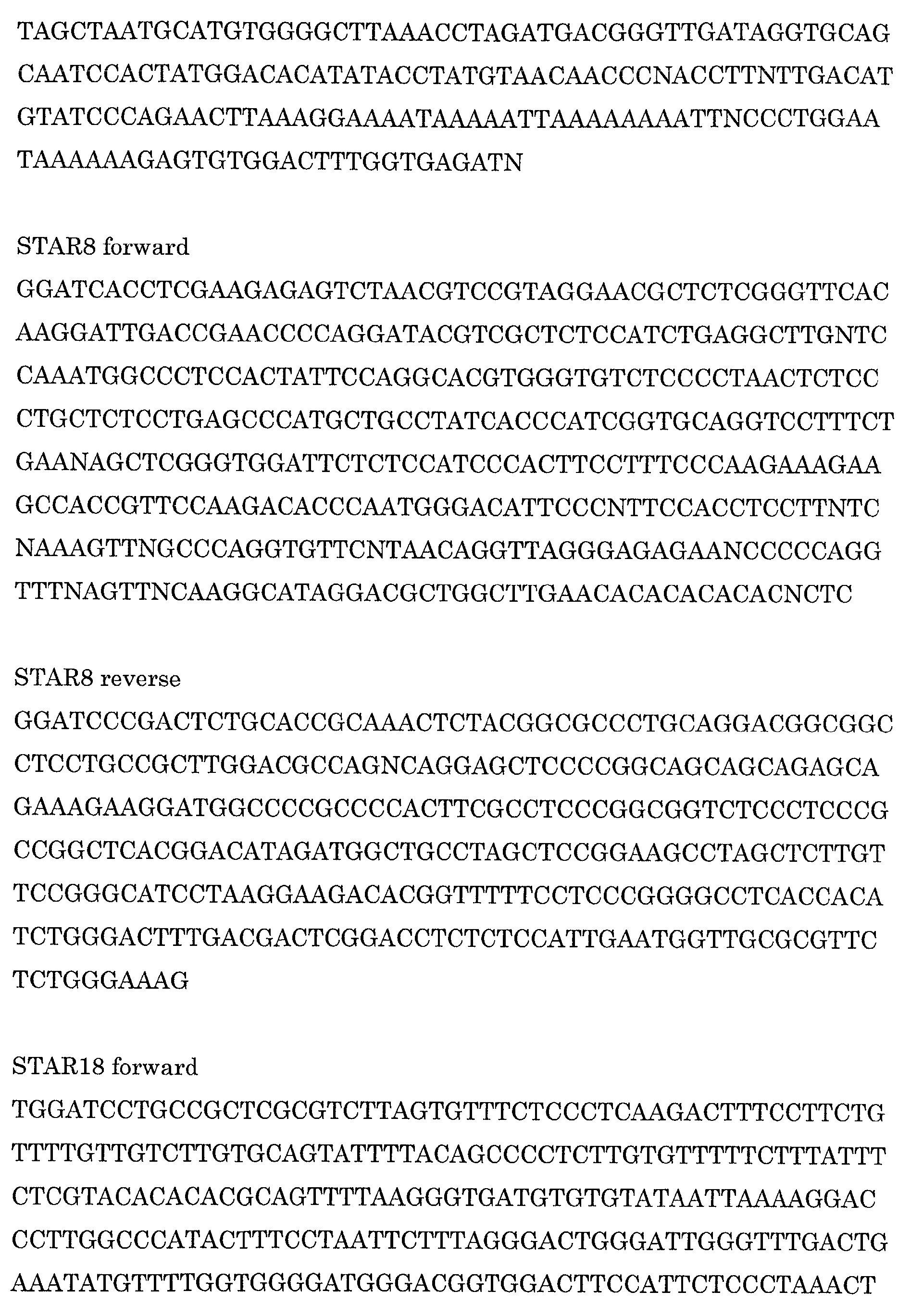

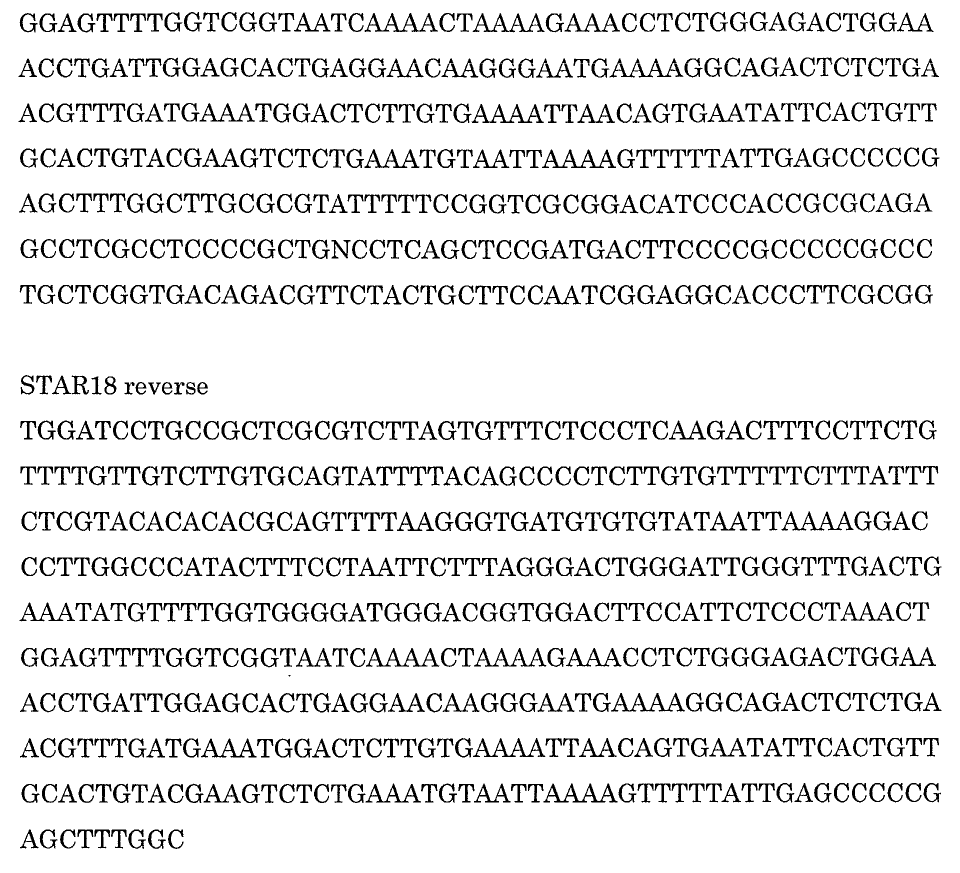

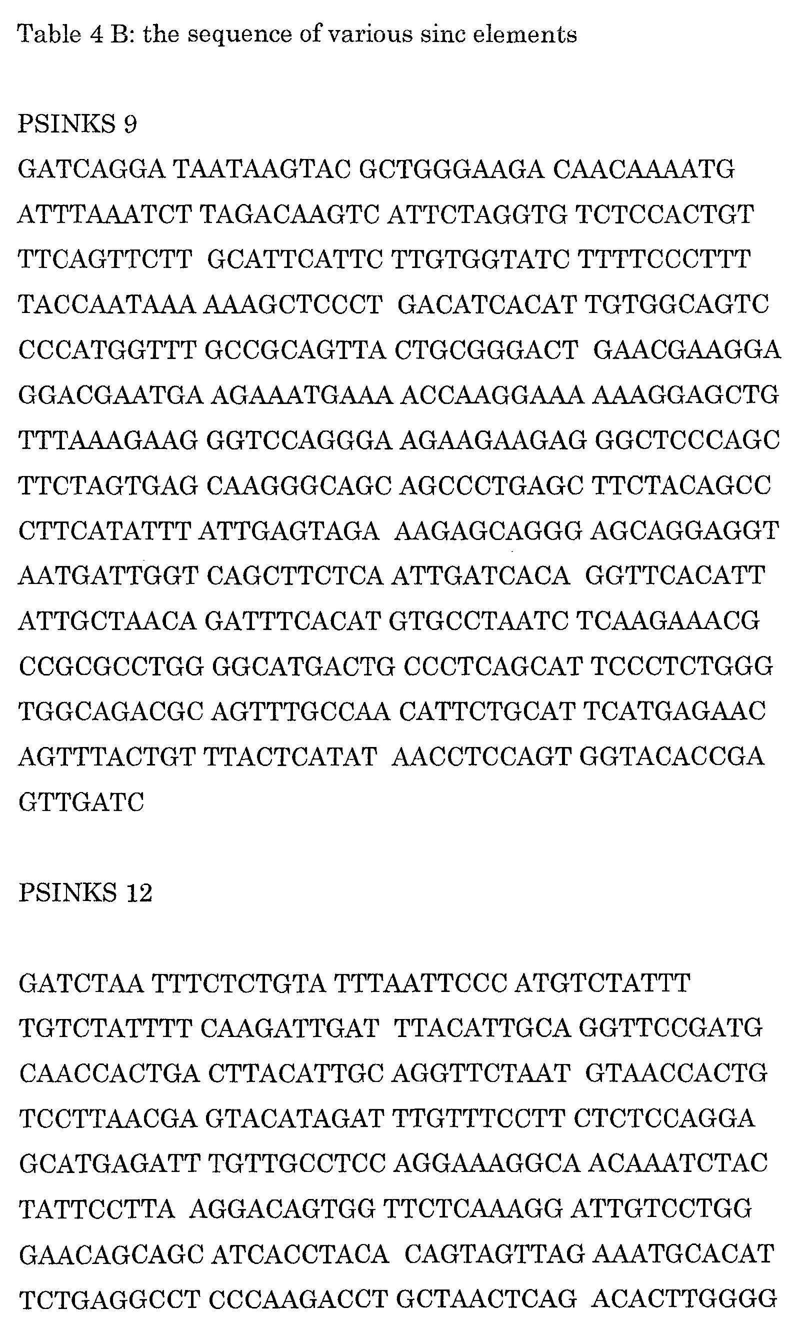

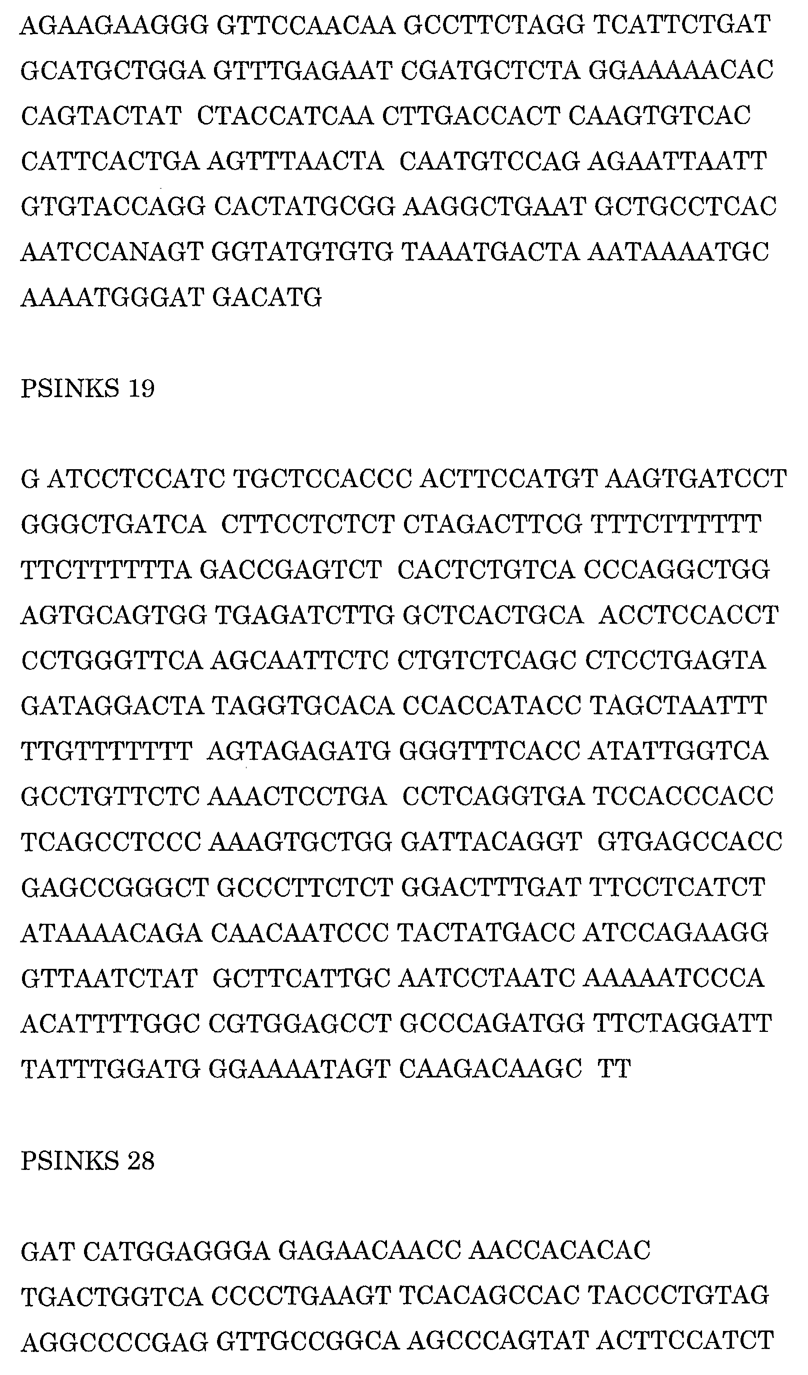

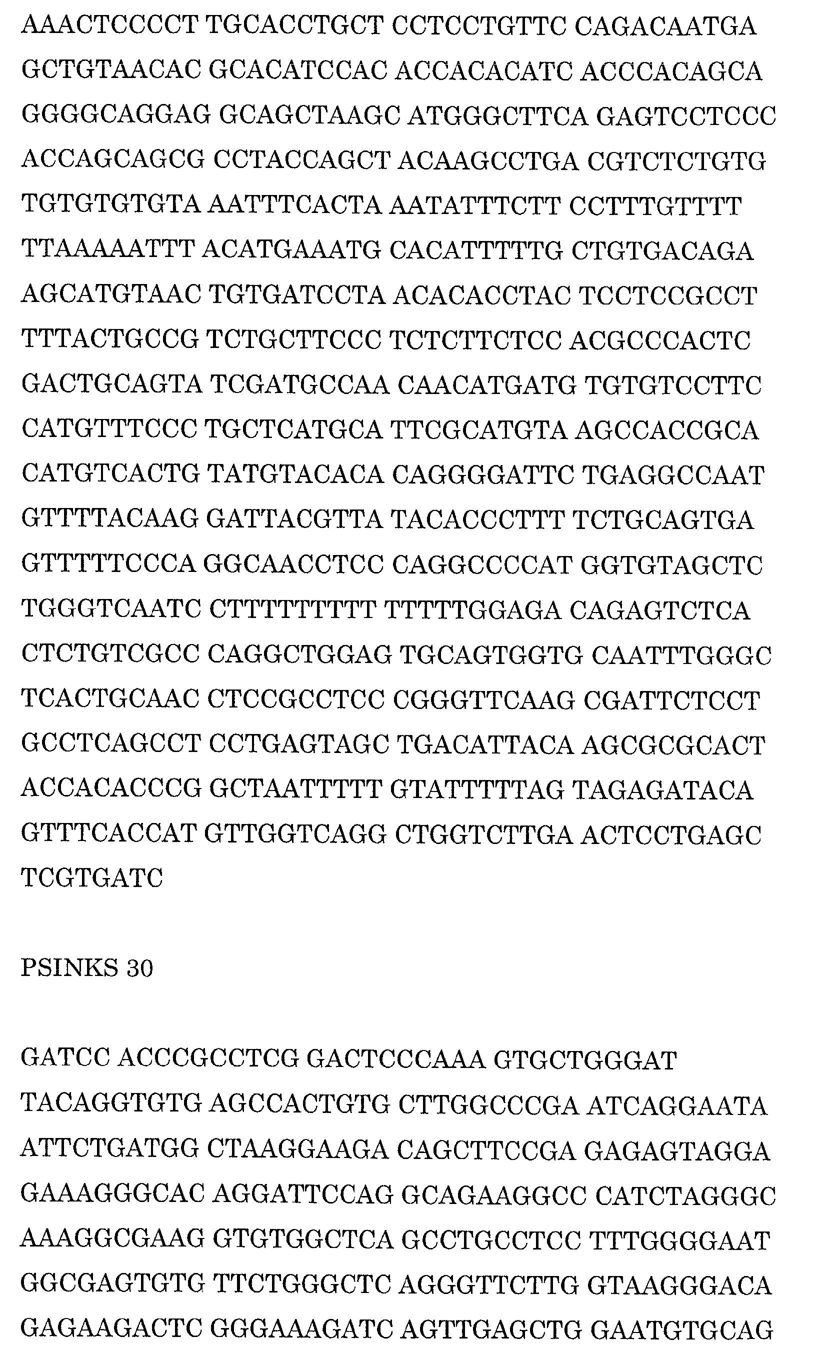

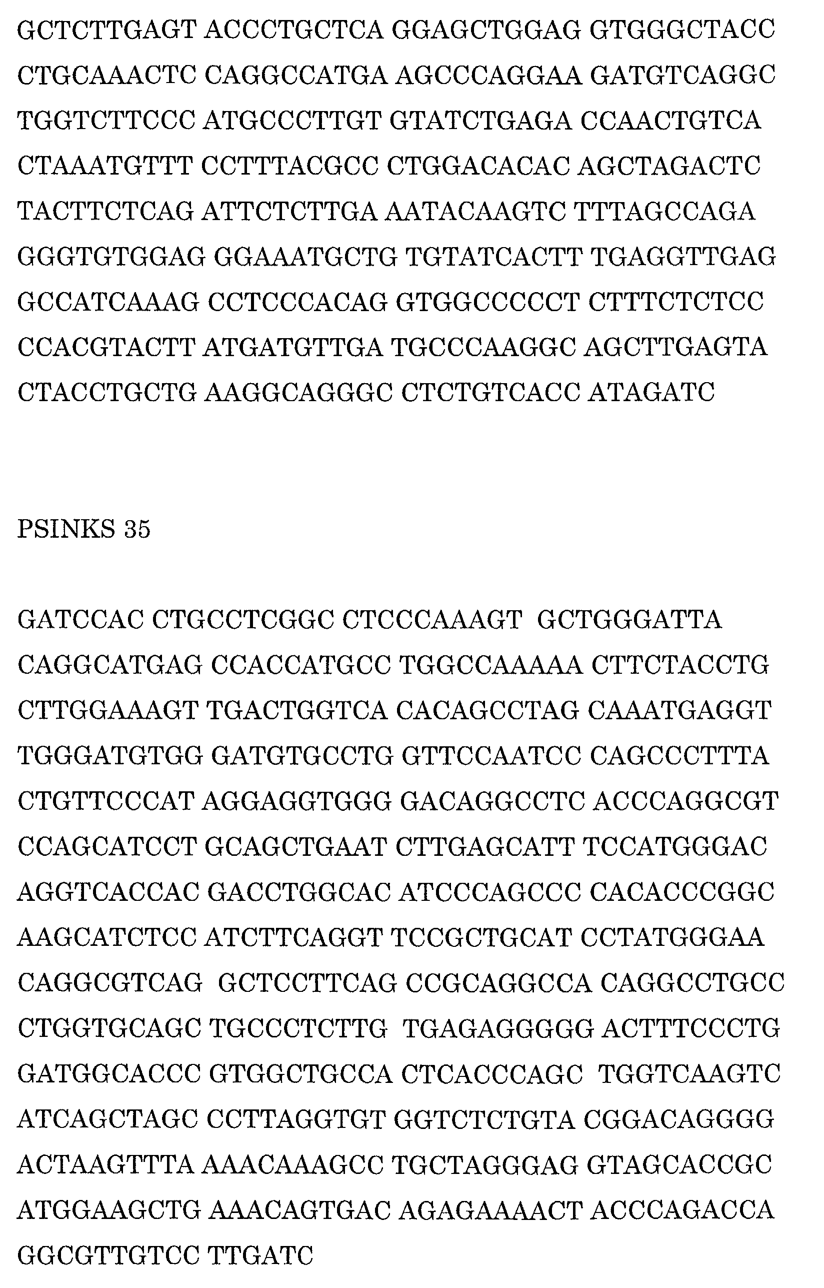

## Claims

1. A method for selecting a DNA sequence with a gene transcription modulating quality, comprising providing a transcription system with a variety of fragment-comprising vectors, said vectors further comprising i) an element with a gene-transcription repressing quality, and ii) a promoter directing transcription of a reporter gene, the method further comprising performing a selection step in said transcription system in order to identify a fragment comprising said DNA sequence with said gene transcription modulating quality.

2. A method according to claim 1, wherein said DNA sequence comprises a stable gene transcription modulating quality.

3. A method according to claim 1 or claim 2, wherein said DNA sequence comprises a gene transcription enhancing quality.

4. A method according to any one of claims 1-3, wherein said transcription system comprises host cells.

5. A method according to any one of claims 1-4, wherein said promoter may be active in said transcription system but wherein gene-transcription repression in said vectors results in gene-transcription repressing chromatin.

6. A method according to any of the preceding claims, wherein the DNA sequence involved in gene-transcription repression is a DNA sequence that is recognized by a protein complex and wherein said transcription system comprises said complex.

7. A method according to claim 5, wherein said complex comprises a heterochromatin-binding protein HP1, a Polycomb-group (Pc-G) protein, a histone deacetylase activity or MeCP2 (methyl-CpG-binding protein.

8. A method according to any of the preceding claims, wherein the vector is an episomally replicating vector.

9. A method according to any of the preceding claims, **characterized in that** the vector comprises a replication origin from the Epstein-Barr virus (EBV), OriP, and a nuclear antigen (EBNA-1).

10. A DNA sequence comprising i) a DNA sequence isolated from a plant or vertebrate, or derivatives thereof, or ii) a synthetic DNA sequence or one constructed by means of genetic engineering, which DNA sequence is a repression-inhibiting sequence which, by means of a method according to any one of claims 1-9, can be detected, selected and optionally cloned.

11. A DNA sequence comprising i) a DNA sequence isolated from a plant or vertebrate, or derivatives thereof, or ii) a synthetic DNA sequence or one constructed by means of genetic engineering, which DNA sequence is detected, selected and optionally cloned by means of the method according to any of the claims 1 to 9.

12. A method for identifying a DNA sequence with a gene transcription repressing quality comprising,
- providing a collection of test nucleic acids,
- generating a collection of expression vectors comprising test nucleic acid and a first reporter gene under transcriptional control of a promoter,
- providing cells with said collection of expression vectors,
- selecting a cell or vector containing progeny thereof, wherein transcription of said first reporter gene is repressed, and
- identifying said test nucleic acid in said cell.

13. A method according to claim 12, wherein said DNA sequence comprises a gene transcription repressing chromatin quality.

14. A method according to claim 12 or claim 13, wherein said DNA sequence comprises a polycomb-group-like responsive element.

15. A method according to any one of claims 12-14, wherein said vectors further comprise a second reporter gene.

16. A method according to claim any one of claims 12-15, wherein said transcription system comprises cells.

17. A method according to claim 16, wherein said second reporter gene is used to select for vector-containing cells.

18. A method according to any one of claims 12-17, wherein vectors further comprise a DNA sequence with a gene transcription modulating quality.

19. A method according to claim 18, wherein said DNA sequence comprises a stable gene transcription modulating quality.

20. A method according to claim 18 or claim 19, wherein said DNA sequence comprises a sequence according to claim 10 or claim 11.

21. A method according to any one of claims 18-20, wherein said DNA sequence at least in part blocks spreading of a transcription repressing effect of a DNA sequence with a gene transcription repressing quality, present on one side of said DNA sequence in said vector.

22. A method according to claim 21, wherein said DNA sequence at least in part inhibits a gene transcription repressing effect on transcription of said second reporter gene.

23. A method according to any one of claims 12-22, wherein said first reporter gene comprises a suicide gene.

24. A method according to any one of claims (12-23), wherein said second reporter gene comprises a dominant selectable reporter gene.

25. A method according to anyone of claims 1-9 or 12-24, wherein said promoter comprises an inducible promoter.

26. A DNA sequence comprising a gene-transcription repressing quality obtainable by a method according to any one of claims 12-25

27. A DNA sequence according to claim 26 comprising a sequence according to table 4 B, or a functional homologue thereof.

28. A method according to any one of claims 1-9, wherein said DNA sequence with a gene transcription repressing quality comprises a DNA sequence obtainable by a method according to any one of claims 12-25.

29. A DNA construct provided with a DNA sequence according to claim 10, 11, 26 or 27.

30. A DNA construct wherein a DNA sequence according to claim 10, 11, 26 or 27 has been modified.

31. A DNA construct according to claim 29 or claim 30, comprising a promoter operably linked with a nucleic acid of interest.

32. A DNA construct according to claim 31, wherein the amount of activity of a quality of said DNA sequence with a gene transcription modulating and/or repressing quality, is dependent on the orientation of said DNA sequence in said construct, compared to said promoter.

33. A DNA construct according to any one of claims 29-32, or a DNA sequence according to claim 10, 11, 26 or 27, wherein a gene transcription modulating and/or repressing quality is dependent on the presence of a signal .

34. A DNA construct or sequence according to claim 33, wherein said signal comprises a DNA binding protein.

35. A DNA construct or sequence according to claim 33 or claim 34, wherein said signal comprises a human immuno-deficiency virus TAT protein.

36. Use of a DNA construct or sequence according to any one of claims 10, 11, 26, 27, 29-35 for regulating transcription of a nucleic acid of interest.

37. A method for producing a gene product in a cell comprising generating an expression cassette comprising a gene of interest and a DNA sequence or DNA construct according to any one of claims 10, 11, 26, 27, 29-35, and allowing transcription of said expression cassette in a cell.

38. A method according to claim 32, wherein production of said gene product is inducible.

39. A library of isolated and/or recombinant nucleic acids comprising a gene transcription modulating quality and/or a gene transcription repressing quality.

40. A library according to claim 39, wherein said nucleic acids comprise the same consensus sequence.

41. A library according to claim 39 or claim 40, wherein said gene transcription repressing quality comprises a quality to induce the formation of gene-transcription repressing chromatin.

42. A library according to any one of claims 39-41, wherein said gene transcription modulating quality comprises a stable transcription quality.

43. A library according to claim 42, wherein said gene transcription modulating quality comprises a quality to counteract the formation of gene-transcription repressing chromatin.

44. A library according to any one of claims 39-43, wherein said gene transcription modulating quality comprises a transcription-enhancing quality.

45. A library according to any one of claims 39-44, comprising essentially all DNA sequences with a gene transcription modulating quality of a chromosome.

46. A library according to claim 45, wherein said chromosome comprises chromosome 21 or chromosome 22.

47. A library according to claim 45 or claim 46, comprising essentially all DNA sequences with a gene transcription modulating quality of the nuclear DNA of a cell.

48. A method for selecting a DNA sequence with a gene transcription modulating quality comprising providing a transcription system with a variety of fragment-comprising vectors, said vectors comprising i) an element with a gene transcription modulating quality, and ii) a promoter directing transcription of a reporter gene, the method further comprising performing a selection step in said transcription system in order to identify a fragment comprising said DNA sequence with said gene transcription modulating quality.


SEQUENCE LISTING

<110> Chromagenics B.V.

<120> DNA sequences comprising gene transcription regulatory
      qualities and methods for detecting and using such DNA
      sequences

<130> P56047EP00

<140> 01202581.3
<141> 2001-07-04

<160> 19

<170> PatentIn Ver. 2.1

<210> 1
<211> 13
<212> DNA
<213> Bacteriophage P1

<220>
<221> misc_feature
<222> (1)..(13)
<223> /note="Lox P target"

<400> 1
ataacttcgt ata                                                    13


<210> 2
<211> 13
<212> DNA
<213> Yeast sp

<220>
<221> misc_feature
<222> (1)..(13)
<223> /note="FLP recombinase target"

<400> 2
gaagttccta tac                                                    13


<210> 3
<211> 4
<212> DNA
<213> Artificial Sequence

<220>
<223> Description of Artificial Sequence: recognition
      site of Hpa II and Msp I restriction endonucleases

<220>
<221> misc_feature
<222> (1)..(4)

<400> 3
ccgg                                                              4

<210> 4
<211> 631
<212> DNA
<213> Homo sapiens

<220>
<221> misc_feature
<222> (1)..(631)
<223> /note="Star element STAR3 forward"

<400> 4
acgtnctaag naaaccatta ttatcatgac attaacctat aaaaataggc gtatcacgag 60
gccctttcgt cttcactcga gcggccagct tggatctcga gtactgaaat aggagtaaat 120
ctgaagagca aataagatga gccagaaaac catgaaaaga acagggacta ccagttgatt 180
ccacaaggac attcccaagg tgagaaggcc atatacctcc actacctgaa ccaattctct 240
gtatgcagat ttagcaaggt tataaggtag caaaagatta gacccaagaa aatagagaac 300
ttccaatcca gtaaaaatca tagcaaattt attgatgata acaattgtct ccaaaggaac 360
caggcagagt cgtgctagca gaggaagcac gtgagctgaa aacagccaaa tctgctttgt 420
tttcatgaca caggagcata aagtacacac caccaactga cctattaagg ctgtggtaaa 480
ccgattcata gagagaggtt ctaaatacat tggtccctca taggcaaacc gcagttcact 540
ccgaacgtag tccctggaaa tttgatgtcc agnatagaaa agcanagcag ncnnnnnnta 600
tanatnnngn tganccanat gntnnctgnn c 631


<210> 5
<211> 774
<212> DNA
<213> Homo sapiens

<220>
<221> misc_feature
<222> (1)..(774)
<223> /note="Star element STAR3 reverse"

<400> 5
gagctagcgg cgcgccaagc ttggatcccg ccccgccccc tccgccctcg agccccgccc 60
cttgccctag aggccctgcc gaggggcggg gcctgtccct cctccccttt cccccgcccc 120
ctaccgtcac gctcaggggc agcctgaccc cgagcggccc cgcggtgacc ctcgcgcaga 180
ggcctgtggg aggggcgtcg caagcccctg aatccccccc cgtctgttcc cccctcccgc 240
ccagtctcct ccccctggga acgcgcgggg tgggtgacag acctggctgc gcgccaccgc 300
caccgcgcct gccgggggcg ctgccgctgc ctgagaaact gcggctgccg cctggaggag 360
gtgccgtcgc ctccgccacc gctgccgccg ccgccagggg taggagctaa gccgccgcca 420
ttttgtgtcc ccctgttgtt gtcgttgaca tgaatccgac atgacactga ttacagccca 480
atggagtctc attaaacccg agtcgcggtc ccgccccgcc gctgctccat tggaggagac 540
caaagacact taaggccacc cgttggccta cgggtctgtc tgtcacccac tcactaacca 600
ctctgcagcc cattggggca ggttcctgcc ggtcatntcg cttccaataa acacacccct 660
tcgaccccat nattcccccc cttcgggaac cacccccggg ggaggggtcc actggncaat 720
accaattnaa nagaaccgct ngggtccgcc tntttncggg cnccctattg ggtt 774


<210> 6
<211> 717
<212> DNA
<213> Homo sapiens

<220>
<221> misc_feature
<222> (1)..(717)
<223> /note="Star element STAR4 forward"

<400> 6
ggggaggatt cttttggctg ctgagttgag attaggttga gggtagtgaa ggtaaaggca 60
gtgagaccac gtaggggtca ttgcagtaat ccaggctgga gatgatggtg gttcagttgg 120
aatagcagtg catgtgctgt aacaacctca gctgggaagc agtatatgtg gcgttatgac 180
ctcagctgga acagcaatgc atgtggtggt gtaatgaccc cagctgggta gggtgcatgt 240
gatggaacaa cctcagctgg gtagcagtgt acttgataaa atgttggcat actctacatt 300
tgttatgagg gtagtgccat taaatttctc cacaaattgg ttgtcacgta tgagtgaaaa 360
gaggaagtga tggaagactt cagtgctttt ggcctgaata aatagaagac gtcattttca 420
gtaatggaga cagggaagac taangnaggg tggattcagt agagcaggtg ttcagttttg 480
aatatgatga actctgagag aggaaaaact ttttctacct cttagttttt gngnctggac 540
ttaanattaa aggacatang acngaganca gaccaaatnt gcgangtttt tatattttac 600
ttgcngaggg aatttncaag aaaaagaaga cccaanancc attggtcaaa actatntgcc 660
ttttaanaaa aaganaatta caatgganan anaagtgttg nctnggcaaa aattggg 717

<210> 7
<211> 541
<212> DNA
<213> Homo sapiens

<220>
<221> misc_feature
<222> (1)..(541)
<223> /note="Star element STAR4 reverse"

<400> 7
ggattngagc tagcggcgcg ccaagcttgg atcttagaag gacagagtgg ggcatggaaa 60
tgcaccacca gggcagtgca gcttggtcac tgccagctcc nctcatgggc agagggctgg 120
cctcttgcag ccgaccaggc actgagcgcc atcccagggc cctcgccagc cctcagcagg 180
gccaggacac acaagccttt gacttcctcc tgtcactgct gctgccattc ctgttttgtg 240
gtcatcactc cttccctgtc ctcagactgc ccagcactca aggatgtcct gtggtggcat 300
cagaccatat gcccctgaan aggagtgagt tggtgttttt tgccgcgccc anagagctgc 360
tgtcccctga aagatgcaag tgggaatgat gatgntcacc atcntctgac accaagccct 420
ttggatagag gccccaacag tgaggatggg gctgcactgc attgccaagg caactctgtn 480
ntgactgcta cangacantc ccaggacctg ngaagnncta tanatntgat gcnaggcacc 540
t 541

<210> 8
<211> 794
<212> DNA
<213> Homo sapiens

<220>
<221> misc_feature
<222> (1)..(794)
<223> /note="Star element STAR6 forward"

<400> 8
ccaccacaga catcccctct ggcctcctga gtggtttctt cagcacagct tccagagcca 60
aattaaacgt tcactctatg tctatagaca aaaagggttt tgactaaact ctgtgtttta 120
gagagggagt taaatgctgt taacttttta ggggtgggcg agaggaatga caaataacaa 180
cttgtctgaa tgttttacat ttctccccac tgcctcaaga aggttcacaa cgaggtcatc 240
catgataagg agtaagacct cccagccgga ctgtccctcg gcccccagag gacactccac 300
agagatatgc taactggact tggagactgg ctcacactcc agagaaaagc atggagcacg 360
agcgcacaga gcangggcca aggtcccagg gacngaatgt ctaggaggga gattggggtg 420
agggtantct gatgcaatta ctgngcagct caacattcaa gggaggggaa gaaagaaacn 480
gtccctgtaa gtaagttgtn cancagagat ggtaagctcc aaatttnaac tttggctgct 540
ggaaagtttn ngggccnana naanaaacan aaanatttga ggtttanacc cactaacccn 600
tatnantant tattaatacc cctaattana ccttggatan ccttaaaata tcntntnaaa 660
cggaaccctc nttcccnttt nnaaatnnna aaggccattn ngnncnagta aaaatctnnn 720

ttaagnnntg ggcccnaaca aacntnttcc nagacacntt ttttntccng gnatttntaa 780
tttatttcta ancc 794

<210> 9
<211> 379
<212> DNA
<213> Homo sapiens

<220>
<221> misc_feature
<222> (1)..(379)
<223> /note="Star element STAR6 reverse"

<400> 9
atcgtgtcct ttccagggac atggatgaag ctggaagcca tcatcctcag caaactaaca 60
caggaacaga aaaccaaata ccacatgttc tcactcataa gtgggagctg aacagtgaga 120
acacatggac acagggaggg gaacatcaca caccaaggcc tgtctggtgt ggggagggga 180
gggagagcat caggacaaat agctaatgca tgtggggctt aaacctagat gacgggttga 240
taggtgcagc aatccactat ggacacatat acctatgtaa caacccnacc ttnttgacat 300
gtatcccaga acttaaagga aaataaaaat taaaaaaaat tnccctggaa taaaaaagag 360
tgtggacttt ggtgagatn 379

<210> 10
<211> 398
<212> DNA
<213> Homo sapiens

<220>
<221> misc_feature
<222> (1)..(398)
<223> /note="Star element STAR8 forward"

<400> 10
ggatcacctc gaagagagtc taacgtccgt aggaacgctc tcgggttcac aaggattgac 60
cgaaccccag gatacgtcgc tctccatctg aggcttgntc caaatggccc tccactattc 120
caggcacgtg ggtgtctccc ctaactctcc ctgctctcct gagcccatgc tgcctatcac 180
ccatcggtgc aggtcctttc tgaanagctc gggtggattc tctccatccc acttcctttc 240
ccaagaaaga agccaccgtt ccaagacacc caatgggaca ttcccnttcc acctccttnt 300
cnaaagttng cccaggtgtt cntaacaggt tagggagaga ancccccagg tttnagttnc 360
aaggcatagg acgctggctt gaacacacac acacnctc 398

<210> 11
<211> 309
<212> DNA
<213> Homo sapiens

<220>
<221> misc_feature
<222> (1)..(309)
<223> /note="Star element STAR8 reverse"

<400> 11
ggatcccgac tctgcaccgc aaactctacg gcgccctgca ggacggcggc ctcctgccgc 60
ttggacgcca gncaggagct ccccggcagc agcagagcag aaagaaggat ggccccgccc 120
cacttcgcct cccggcggtc tccctcccgc cggctcacgg acatagatgg ctgcctagct 180
ccggaagcct agctcttgtt ccgggcatcc taaggaagac acggtttttc ctcccggggc 240
ctcaccacat ctgggacttt gacgactcgg acctctctcc attgaatggt tgcgcgttct 300
ctgggaaag 309

<210> 12
<211> 606
<212> DNA
<213> Homo sapiens

<220>
<221> misc_feature
<222> (1)..(606)
<223> /note="Star element STAR18 forward"

<400> 12
tggatcctgc cgctcgcgtc ttagtgtttc tccctcaaga ctttccttct gttttgttgt 60
cttgtgcagt attttacagc ccctcttgtg tttttcttta tttctcgtac acacacgcag 120
ttttaagggt gatgtgtgta taattaaaag gacccttggc ccatactttc ctaattcttt 180
agggactggg attgggtttg actgaaatat gttttggtgg ggatgggacg gtggacttcc 240
attctcccta aactggagtt ttggtcggta atcaaaacta aaagaaacct ctgggagact 300
ggaaacctga ttggagcact gaggaacaag ggaatgaaaa ggcagactct ctgaacgttt 360
gatgaaatgg actcttgtga aaattaacag tgaatattca ctgttgcact gtacgaagtc 420
tctgaaatgt aattaaaagt ttttattgag cccccgagct ttggcttgcg cgtatttttc 480
cggtcgcgga catcccaccg cgcagagcct cgcctccccg ctgncctcag ctccgatgac 540
ttccccgccc ccgccctgct cggtgacaga cgttctactg cttccaatcg gaggcaccct 600
tcgcgg 606


<210> 13
<211> 465
<212> DNA
<213> Homo sapiens

<220>
<221> misc_feature
<222> (1)..(465)
<223> /note="Star element STAR18 reverse"

<400> 13
tggatcctgc cgctcgcgtc ttagtgtttc tccctcaaga ctttccttct gttttgttgt 60
cttgtgcagt attttacagc ccctcttgtg tttttcttta tttctcgtac acacacgcag 120
ttttaagggt gatgtgtgta taattaaaag gacccttggc ccatactttc ctaattcttt 180
agggactggg attgggtttg actgaaatat gttttggtgg ggatgggacg gtggacttcc 240
attctcccta aactggagtt ttggtcggta atcaaaacta aaagaaacct ctgggagact 300
ggaaacctga ttggagcact gaggaacaag ggaatgaaaa ggcagactct ctgaacgttt 360
gatgaaatgg actcttgtga aaattaacag tgaatattca ctgttgcact gtacgaagtc 420
tctgaaatgt aattaaaagt ttttattgag cccccgagct ttggc 465


<210> 14
<211> 565
<212> DNA
<213> Homo sapiens

<220>
<221> misc_feature
<222> (1)..(565)
<223> /note="Sinc element PSINKS 9"

<400> 14
gatcaggata ataagtacgc tgggaagaca acaaaatgat ttaaatctta gacaagtcat 60
tctaggtgtc tccactgttt cagttcttgc attcattctt gtggtatctt ttccctttta 120
ccaataaaaa agctccctga catcacattg tggcagtccc catggtttgc cgcagttact 180

gcgggactga acgaaggagg acgaatgaag aaatgaaaac caaggaaaaa aggagctgtt 240
taaagaaggg tccagggaag aagaagaggg ctcccagctt ctagtgagca agggcagcag 300
ccctgagctt ctacagccct tcatatttat tgagtagaaa gagcagggag caggaggtaa 360
tgattggtca gcttctcaat tgatcacagg ttcacattat tgctaacaga tttcacatgt 420
gcctaatctc aagaaacgcc gcgcctgggg catgactgcc ctcagcattc cctctgggtg 480
gcagacgcag tttgccaaca ttctgcattc atgagaacag tttactgttt actcatataa 540
cctccagtgg tacaccgagt tgatc 565

<210> 15
<211> 570
<212> DNA
<213> Homo sapiens

<220>
<221> misc_feature
<222> (1)..(570)
<223> /note="Sinc element PSINKS 12"

<400> 15
gatctaattt ctctgtattt aattcccatg tctattttgt ctattttcaa gattgattta 60
cattgcaggt tccgatgcaa ccactgactt acattgcagg ttctaatgta accactgtcc 120
ttaacgagta catagatttg tttccttctc tccaggagca tgagatttgt tgcctccagg 180
aaaggcaaca aatctactat tcctaaggac agtggttctc aaaggattgt cctgggaaca 240
gcagcatcac ctacacagta gttagaaatg cacattctga ggcctcccaa gacctgctaa 300
ctcagacact tggggagaag aaggggttcc aacaagcctt ctaggtcatt ctgatgcatg 360
ctggagtttg agaatcgatg ctctaggaaa aacaccagta ctatctacca tcaacttgac 420
cactcaagtg tcaccattca ctgaagttta actacaatgt ccagagaatt aattgtgtac 480
caggcactat gcggaaggct gaatgctgcc tcacaatcca nagtggtatg tgtgtaaatg 540
actaaataaa atgcaaaatg ggatgacatg 570

<210> 16
<211> 553
<212> DNA
<213> Homo sapiens

<220>
<221> misc_feature
<222> (1)..(553)
<223> /note="Sinc element PSINKS 19

<400> 16
gatcctccat ctgctccacc cacttccatg taagtgatcc tgggctgatc acttcctctc 60
tctagacttc gtttcttttt tttctttttt agaccgagtc tcactctgtc acccaggctg 120
gagtgcagtg gtgagatctt ggctcactgc aacctccacc tcctgggttc aagcaattct 180
cctgtctcag cctcctgagt agataggact ataggtgcac accaccatac ctagctaatt 240
tttgtttttt tagtagagat ggggtttcac catattggtc agcctgttct caaactcctg 300
acctcaggtg atccacccac ctcagcctcc caaagtgctg ggattacagg tgtgagccac 360
cgagccgggc tgcccttctc tggactttga tttcctcatc tataaaacag acaacaatcc 420
ctactatgac catccagaag ggttaatcta tgcttcattg caatcctaat caaaaatccc 480
aacatttggg ccgtggagcc tgcccagatg gttctaggat ttatttggat gggaaaatag 540
tcaagacaag ctt 553

<210> 17
<211> 881
<212> DNA
<213> Homo sapiens

<220>

<221> misc_feature
<222> (1)..(881)
<223> /note="Sinc element PSINKS 28"

<400> 17
gatcatggag ggagagaaca accaaccaca cactgactgg tcacccctga agttcacagc 60
cactaccctg tagaggcccc gaggttgccg gcaagcccag tatacttcca tctaaactcc 120
ccttgcacct gctcctcctg ttccagacaa tgagctgtaa cacgcacatc cacaccacac 180
atcacccaca gcaggggcag gaggcagcta agcatgggct tcagagtcct cccaccagca 240
gcgcctacca gctacaagcc tgacgtctct gtgtgtgtgt gtaaatttca ctaaatattt 300
cttcctttgt tttttaaaaa tttacatgaa atgcacattt ttgctgtgac agaagcatgt 360
aactgtgatc ctaacacacc tactcctccg ccttttactg ccgtctgctt ccctctcttc 420
tccacgccca ctcgactgca gtatcgatgc caacaacatg atgtgtgtcc ttccatgttt 480
ccctgctcat gcattcgcat gtaagccacc gcacatgtca ctgtatgtac acacagggga 540
ttctgaggcc aatgttttac aaggattacg ttatacaccc ttttctgcag tgagtttttc 600
ccaggcaacc tcccaggccc catggtgtag ctctgggtca atcctttttt tttttttggg 660
agacagagtc tcactctgtc gcccaggctg gagtgcagtg gtgcaatttg ggctcactgc 720
aacctccgcc tcccgggttc aagcgattct cctgcctcag cctcctgagt agctgacatt 780
acaagcgcgc actaccacac ccggctaatt tttgtatttt tagtagagat acagtttcac 840
catgttggtc aggctggtct tgaactcctg agctcgtgat c 881


<210> 18
<211> 632
<212> DNA
<213> Homo sapiens

<220>
<221> misc_feature
<222> (1)..(632)
<223> /note="Sinc element PSINKS 30"

<400> 18
gatccacccg cctcggactc ccaaagtgct gggattacag gtgtgagcca ctgtgcttgg 60
cccgaatcag gaataattct gatggctaag gaagacagct tccgagagag taggagaaag 120
ggcacaggat tccaggcaga aggcccatct agggcaaagg cgaaggtgtg gctcagcctg 180
cctcctttgg ggaatggcga gtgtgttctg ggctcagggt tcttggtaag ggacagagaa 240
gactcgggaa agatcagttg agctggaatg tgcaggctct tgagtaccct gctcaggagc 300
tggaggtggg ctaccctgca aactccaggc catgaagccc aggaagatgt caggctggtc 360
ttcccatgcc cttgtgtatc tgagaccaac tgtcactaaa tgtttccttt acgccctgga 420
cacacagcta gactctactt ctcagattct cttgaaatac aagtctttag ccagagggtg 480
tggagggaaa tgctgtgtat cactttgagg ttgaggccat caaagcctcc cacaggtggc 540
cccctctttc tctccccacg tacttatgat gttgatgccc aaggcagctt gagtactacc 600
tgctgaaggc agggcctctg tcaccataga tc 632


<210> 19
<211> 573
<212> DNA
<213> Homo sapiens

<220>
<221> misc_feature
<222> (1)..(573)
<223> /note="Sinc element PSINKS 35"

<400> 19
gatccacctg cctcggcctc ccaaagtgct gggattacag gcatgagcca ccatgcctgg 60
ccaaaaactt ctacctgctt ggaaagttga ctggtcacac agcctagcaa atgaggttgg 120
gatgtgggat gtgcctggtt ccaatcccag ccctttactg ttcccatagg aggtggggac 180
aggcctcacc caggcgtcca gcatcctgca gctgaatctt gagcatttcc atgggacagg 240

tcaccacgac ctggcacatc ccagccccac acccggcaag catctccatc ttcaggttcc 300
gctgcatcct atgggaacag gcgtcaggct ccttcagccg caggccacag gcctgccctg 360
gtgcagctgc cctcttgtga gagggggact ttccctggat ggcacccgtg gctgccactc 420
acccagctgg tcaagtcatc agctagccct taggtgtggt ctctgtacgg acaggggact 480
aagtttaaaa caaagcctgc tagggaggta gcaccgcatg gaagctgaaa cagtgacaga 540
gaaaactacc cagaccaggc gttgtccttg atc 573